# EUROPEAN PATENT SPECIFICATION

(11) **EP 2 829 056 B1**
(45) Date of publication and mention of the grant of the patent: **16.10.2019**
(21) Application number: 13770520.8
(22) Date of filing: 14.03.2013
(51) Int. Cl.: G08B 21/12, H04N 5/33, H04N 7/18

(54) **WEARABLE APPARATUS WITH INTEGRATED INFRARED IMAGING MODULE**
TRAGBARE VORRICHTUNG MIT EINEM INTEGRIERTEN INFRAROTBILDGEBUNGSMODUL
APPAREIL PORTABLE À MODULE D'IMAGERIE INFRAROUGE INTÉGRÉ

(30) Priority: 19.03.2012 US 201261612794 P; 07.06.2012 US 201261656889 P; 08.06.2012 WO PCT/US2012/041749; 08.06.2012 WO PCT/US2012/041739; 08.06.2012 WO PCT/US2012/041744; 13.03.2013 US 201313802615
(43) Date of publication of application: 28.01.2015
(73) Proprietor: Flir Systems, Inc., Goleta, CA 93117 (US)
(72) Inventor: TERRE, William A., Goleta, California 93117 (US); TEICH, Andrew C., Goleta, California 93117 (US); LEPORE,Giovanni, Goleta, California 93117 (US); HÖGASTEN, Nicholas, Goleta, California 93117 (US); HOELTER, Theodore R., Goleta, California 93117 (US); STRANDEMAR, Katrin, SE-76294 Rimbo (SE)
(74) Representative: Kitzler, Michael
(86) International application number: PCT/US2013/031734
(87) International publication number: WO 2013/184220

(56) References cited:
- WO-A1-98/38908
- WO-A1-2005/102230
- CN-A- 101 214 178
- JP-A- 2006 176 931
- US-A- 4 884 137
- US-A- 6 016 160
- US-A- 6 072 445
- US-A1- 2010 088 793
- US-B1- 6 384 982

## Description

### TECHNICAL FIELD

One or more embodiments of the invention relate generally to thermal imaging devices and more particularly, for example, to wearable devices for use with thermal imaging devices.

### BACKGROUND

Various wearable devices have been developed to protect users while in hazardous environments. For example, self-contained breathing apparatus (SCBA) devices are widely utilized by firefighters and other emergency personnel to supply breathable air, as well as to protect their facial areas from heat, flames, debris, and other harmful elements when working in hazardous environments. In another example, welding masks are worn by welders to protect their facial areas from intense light, heat, sparks, and other harmful elements that may be generated during welding.

In these and other conditions, visible light imaging sensors (e.g., CCD-based or CMOS-based sensors) typically cannot capture useful images of surrounding environments when visibility is compromised. Conventional infrared cameras (e.g., used to capture thermal images) may also be unsuitable, because such cameras are typically too bulky and heavy, and are generally handheld or otherwise positioned external to the user. Also, field of view discrepancies and misalignment issues may occur, between where a user may be looking relative to where the conventional infrared camera is pointed, due to the externally mounted infrared camera (e.g., mounted on the helmet of the user) not being completely aligned and tracking precisely the head movements of the user. Moreover, external housings may be required to protect conventional infrared cameras from hazardous external environments. Such housings may add even further bulk and weight, and thus make conventional infrared cameras even more unsuitable for use in hazardous environments.

In addition, it is often difficult for users to view images while engaged in hazardous environments. For example, certain conventional displays (e.g., LCD screens to present images for a user to view directly and/or through a scope) are often problematic when used in hazardous environments. In this regard, external handheld display screens may be unwieldy and may limit the ability of a user to engage in activities. If provided within a mask of a wearable device, a conventional display may actually obstruct a user's view and may make it difficult for the user to adjust the screen position or to simultaneously view the surrounding external environment. Moreover, the mounting of conventional displays (e.g., screens, scopes, and/or eyepieces) at the outer periphery of a user's mask may adversely shift the center of gravity of the wearable device forward which may encumber and fatigue the user.

Documents WO-A1-98/38908, US-A-6016160, WO-A1-2005/102230, CN-A-101214178, US-A-4884137, US-A-6072445, JP-A-2006/176931, US-A-2010/0088793 and US-B1-6384982 describe some of such personal wearable protection apparatuses provided with image vision system.

### SUMMARY

Various techniques are disclosed for providing a wearable apparatus having a shield, an infrared imaging module, and a projector to present a user-viewable thermal image of an external environment on a surface of the shield. For example, a self-contained breathing apparatus (SCBA) may include a shield to protect a user from an external environment, one or more infrared imaging modules, a projector, a processor, and a communication module for projecting a user-viewable thermal image onto a surface of the shield. Such infrared imaging modules may be positioned internal to the SCBA so that they are also protected from the external environment, such as a hazardous environment. In another example, a welding mask may include one or more infrared imaging modules, a projector, a processor, and a communication module, so as to project a user-viewable thermal image onto a surface of a shield of the welding mask, while at the same time protecting these components and the welder's face from a harsh welding environment.

In one embodiment, a wearable apparatus is provided as defined in the appended independent claim 1.

In another embodiment, a method of operating a wearable apparatus is provided as defined in the appended independent claim 7.

In another embodiment, a method of constructing a wearable apparatus is provided as defined in the appended independent claim 12.

Advantageous embodiments are defined in the appended dependent claims.

The scope of the invention is defined by the claims, which are incorporated into this section by reference. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### BRIEF DESCRIPTION OF THE DRAWINGS

Fig. 1 illustrates an infrared imaging module configured to be implemented in a host device in accordance with an embodiment of the disclosure.
Fig. 2 illustrates an assembled infrared imaging module in accordance with an embodiment of the disclosure.
Fig. 3 illustrates an exploded view of an infrared imaging module juxtaposed over a socket in accordance with an embodiment of the disclosure.
Fig. 4 illustrates a block diagram of an infrared sensor assembly including an array of infrared sensors in accordance with an embodiment of the disclosure.
Fig. 5 illustrates a flow diagram of various operations to determine non-uniformity correction (NUC) terms in accordance with an embodiment of the disclosure.
Fig. 6 illustrates differences between neighboring pixels in accordance with an embodiment of the disclosure.
Fig. 7 illustrates a flat field correction technique in accordance with an embodiment of the disclosure.
Fig. 8 illustrates various image processing techniques of Fig. 5 and other operations applied in an image processing pipeline in accordance with an embodiment of the disclosure.
Fig. 9 illustrates a temporal noise reduction process in accordance with an embodiment of the disclosure.
Fig. 10 illustrates particular implementation details of several processes of the image processing pipeline of Fig. 8 in accordance with an embodiment of the disclosure.
Fig. 11 illustrates spatially correlated fixed pattern noise (FPN) in a neighborhood of pixels in accordance with an embodiment of the disclosure.
Fig. 12 illustrates a block diagram of a wearable apparatus in accordance with an embodiment of the disclosure.
Figs. 13A-C illustrate various views of a wearable apparatus implemented as a self-contained breathing apparatus (SCBA) in accordance with embodiments of the disclosure.
Figs. 14A-B illustrate side views of several wearable apparatuses implemented as welding masks in accordance with various embodiments of the disclosure.
Fig. 15 illustrates a process to present a user-viewable thermal image on a wearable apparatus in accordance with an embodiment of the disclosure.

Embodiments of the invention and their advantages are best understood by referring to the detailed description that follows. It should be appreciated that like reference numerals are used to identify like elements illustrated in one or more of the figures.

### DETAILED DESCRIPTION

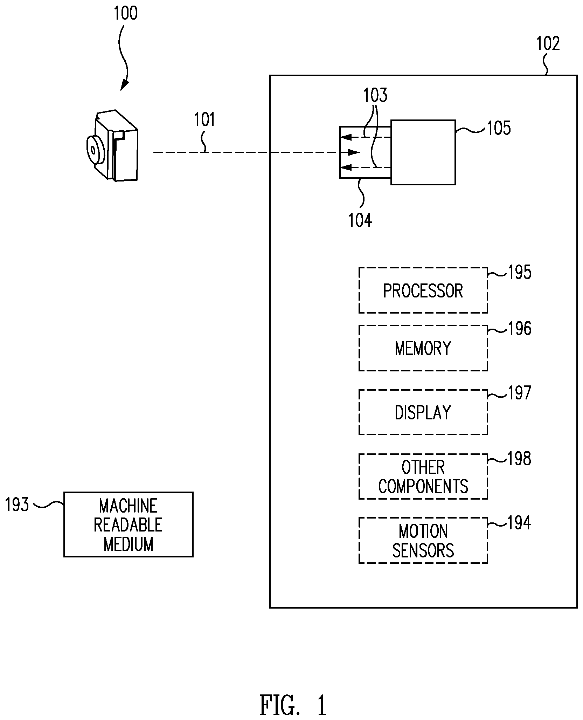

Fig. 1 illustrates an infrared imaging module 100 (e.g., an infrared camera or an infrared imaging device) configured to be implemented in a host device 102 in accordance with an example of the disclosure, not belonging to the present invention. Infrared imaging module 100 may be implemented, for one or more embodiments, with a small form factor and in accordance with wafer level packaging techniques or other packaging techniques.

In one example, infrared imaging module 100 may be configured to be implemented in a small portable host device 102, such as a mobile telephone, a tablet computing device, a laptop computing device, a personal digital assistant, a visible light camera, a music player, or any other appropriate mobile device. In this regard, infrared imaging module 100 may be used to provide infrared imaging features to host device 102. For example, infrared imaging module 100 may be configured to capture, process, and/or otherwise manage infrared images and provide such infrared images to host device 102 for use in any desired fashion (e.g., for further processing, to store in memory, to display, to use by various applications running on host device 102, to export to other devices, or other uses).

In various examples not belonging to the present invention, infrared imaging module 100 may be configured to operate at low voltage levels and over a wide temperature range. For example, in one example, infrared imaging module 100 may operate using a power supply of approximately 2.4 volts, 2.5 volts, 2.8 volts, or lower voltages, and operate over a temperature range of approximately -20 degrees C to approximately +60 degrees C (e.g., providing a suitable dynamic range and performance over an environmental temperature range of approximately 80 degrees C). In one example, by operating infrared imaging module 100 at low voltage levels, infrared imaging module 100 may experience reduced amounts of self heating in comparison with other types of infrared imaging devices. As a result, infrared imaging module 100 may be operated with reduced measures to compensate for such self heating.

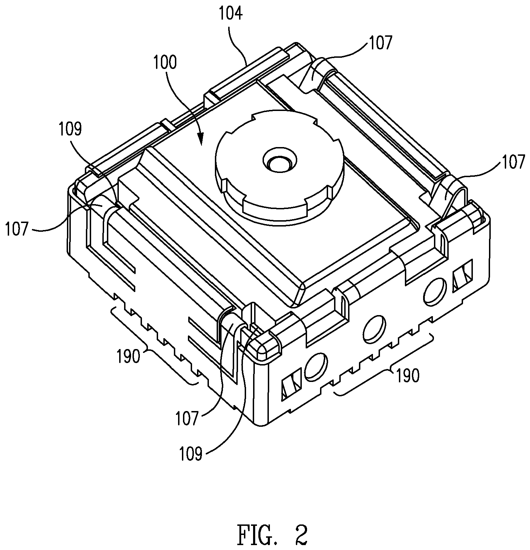

As shown in Fig. 1, host device 102 may include a socket 104, a shutter 105, motion sensors 194, a processor 195, a memory 196, a display 197, and/or other components 198. Socket 104 may be configured to receive infrared imaging module 100 as identified by arrow 101. In this regard, Fig. 2 illustrates infrared imaging module 100 assembled in socket 104 in accordance with an example of the disclosure.

Motion sensors 194 may be implemented by one or more accelerometers, gyroscopes, or other appropriate devices that may be used to detect movement of host device 102. Motion sensors 194 may be monitored by and provide information to processing module 160 or processor 195 to detect motion. In various examples, motion sensors 194 may be implemented as part of host device 102 (as shown in Fig. 1), infrared imaging module 100, or other devices attached to or otherwise interfaced with host device 102.

Processor 195 may be implemented as any appropriate processing device (e.g., logic device, microcontroller, processor, application specific integrated circuit (ASIC), or other device) that may be used by host device 102 to execute appropriate instructions, such as software instructions provided in memory 196. Display 197 may be used to display captured and/or processed infrared images and/or other images, data, and information. Other components 198 may be used to implement any features of host device 102 as may be desired for various applications (e.g., clocks, temperature sensors, a visible light camera, or other components). In addition, a machine readable medium 193 may be provided for storing non-transitory instructions for loading into memory 196 and execution by processor 195.

In various examples, infrared imaging module 100 and socket 104 may be implemented for mass production to facilitate high volume applications, such as for implementation in mobile telephones or other devices (e.g., requiring small form factors). In one embodiment, the combination of infrared imaging module 100 and socket 104 may exhibit overall dimensions of approximately 8.5 mm by 8.5 mm by 5.9 mm while infrared imaging module 100 is installed in socket 104.

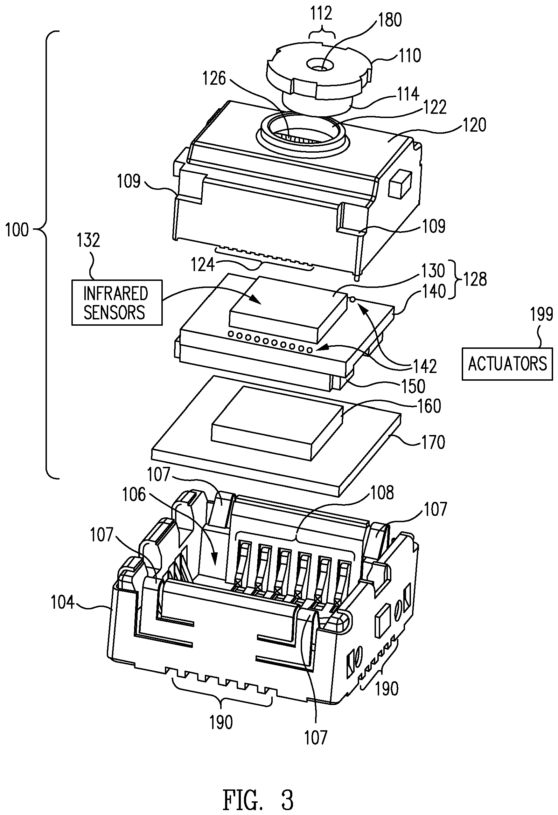

Fig. 3 illustrates an exploded view of infrared imaging module 100 juxtaposed over socket 104 in accordance with an example of the disclosure. Infrared imaging module 100 may include a lens barrel 110, a housing 120, an infrared sensor assembly 128, a circuit board 170, a base 150, and a processing module 160.

Lens barrel 110 may at least partially enclose an optical element 180 (e.g., a lens) which is partially visible in Fig. 3 through an aperture 112 in lens barrel 110. Lens barrel 110 may include a substantially cylindrical extension 114 which may be used to interface lens barrel 110 with an aperture 122 in housing 120.

Infrared sensor assembly 128 may be implemented, for example, with a cap 130 (e.g., a lid) mounted on a substrate 140. Infrared sensor assembly 128 may include a plurality of infrared sensors 132 (e.g., infrared detectors) implemented in an array or other fashion on substrate 140 and covered by cap 130. For example, in one embodiment, infrared sensor assembly 128 may be implemented as a focal plane array (FPA). Such a focal plane array may be implemented, for example, as a vacuum package assembly (e.g., sealed by cap 130 and substrate 140). In one example, infrared sensor assembly 128 may be implemented as a wafer level package (e.g., infrared sensor assembly 128 may be singulated from a set of vacuum package assemblies provided on a wafer). In one example, infrared sensor assembly 128 may be implemented to operate using a power supply of approximately 2.4 volts, 2.5 volts, 2.8 volts, or similar voltages.

Infrared sensors 132 may be configured to detect infrared radiation (e.g., infrared energy) from a target scene including, for example, mid wave infrared wave bands (MWIR), long wave infrared wave bands (LWIR), and/or other thermal imaging bands as may be desired in particular implementations. In one example, infrared sensor assembly 128 may be provided in accordance with wafer level packaging techniques.

Infrared sensors 132 may be implemented, for example, as microbolometers or other types of thermal imaging infrared sensors arranged in any desired array pattern to provide a plurality of pixels. In one example, infrared sensors 132 may be implemented as vanadium oxide (VOx) detectors with a 17 µm pixel pitch. In various examples, arrays of approximately 32 by 32 infrared sensors 132, approximately 64 by 64 infrared sensors 132, approximately 80 by 64 infrared sensors 132, or other array sizes may be used.

Substrate 140 may include various circuitry including, for example, a read out integrated circuit (ROIC) with dimensions less than approximately 5.5 mm by 5.5 mm in one example, Substrate 140 may also include bond pads 142 that may be used to contact complementary connections positioned on inside surfaces of housing 120 when infrared imaging module 100 is assembled as shown in Fig. 3. In one example, the ROIC may be implemented with low-dropout regulators (LDO) to perform voltage regulation to reduce power supply noise introduced to infrared sensor assembly 128 and thus provide an improved power supply rejection ratio (PSRR). Moreover, by implementing the LDO with the ROIC (e.g., within a wafer level package), less die area may be consumed and fewer discrete die (or chips) are needed.

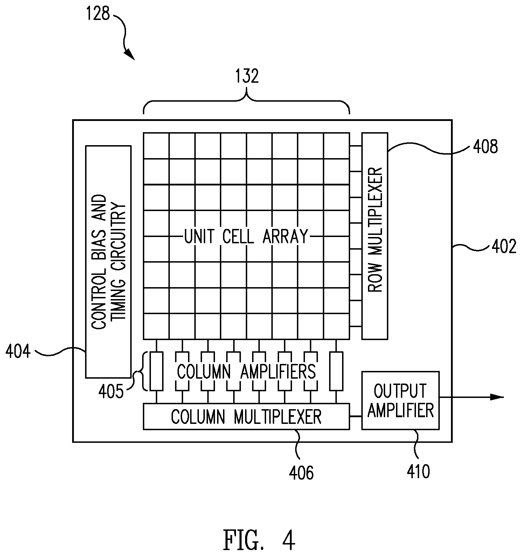

Fig. 4 illustrates a block diagram of infrared sensor assembly 128 including an array of infrared sensors 132 in accordance with an example of the disclosure. In the illustrated example, infrared sensors 132 are provided as part of a unit cell array of a ROIC 402. ROIC 402 includes bias generation and timing control circuitry 404, column amplifiers 405, a column multiplexer 406, a row multiplexer 408, and an output amplifier 410. Image frames (e.g., thermal images) captured by infrared sensors 132 may be provided by output amplifier 410 to processing module 160, processor 195, and/or any other appropriate components to perform various processing techniques described herein. Although an 8 by 8 array is shown in Fig. 4, any desired array configuration may be used in other embodiments. Further descriptions of ROICs and infrared sensors (e.g., microbolometer circuits) may be found in U.S. Patent No. 6,028,309 issued February 22, 2000.

Infrared sensor assembly 128 may capture images (e.g., image frames) and provide such images from its ROIC at various rates. Processing module 160 may be used to perform appropriate processing of captured infrared images and may be implemented in accordance with any appropriate architecture. In one example, processing module 160 may be implemented as an ASIC. In this regard, such an ASIC may be configured to perform image processing with high performance and/or high efficiency. In another example, processing module 160 may be implemented with a general purpose central processing unit (CPU) which may be configured to execute appropriate software instructions to perform image processing, coordinate and perform image processing with various image processing blocks, coordinate interfacing between processing module 160 and host device 102, and/or other operations. In yet another example, processing module 160 may be implemented with a field programmable gate array (FPGA). Processing module 160 may be implemented with other types of processing and/or logic circuits in other examples as would be understood by one skilled in the art.

In these and other examples, processing module 160 may also be implemented with other components where appropriate, such as, volatile memory, non-volatile memory, and/or one or more interfaces (e.g., infrared detector interfaces, inter-integrated circuit (I2C) interfaces, mobile industry processor interfaces (MIPI), joint test action group (JTAG) interfaces (e.g., IEEE 1149.1 standard test access port and boundary-scan architecture), and/or other interfaces).

In some examples, infrared imaging module 100 may further include one or more actuators 199 which may be used to adjust the focus of infrared image frames captured by infrared sensor assembly 128. For example, actuators 199 may be used to move optical element 180, infrared sensors 132, and/or other components relative to each other to selectively focus and defocus infrared image frames in accordance with techniques described herein. Actuators 199 may be implemented in accordance with any type of motion-inducing apparatus or mechanism, and may positioned at any location within or external to infrared imaging module 100 as appropriate for different applications.

When infrared imaging module 100 is assembled, housing 120 may substantially enclose infrared sensor assembly 128, base 150, and processing module 160. Housing 120 may facilitate connection of various components of infrared imaging module 100. For example, in one example, housing 120 may provide electrical connections 126 to connect various components as further described.

Electrical connections 126 (e.g., conductive electrical paths, traces, or other types of connections) may be electrically connected with bond pads 142 when infrared imaging module 100 is assembled. In various examples, electrical connections 126 may be embedded in housing 120, provided on inside surfaces of housing 120, and/or otherwise provided by housing 120. Electrical connections 126 may terminate in connections 124 protruding from the bottom surface of housing 120 as shown in Fig. 3. Connections 124 may connect with circuit board 170 when infrared imaging module 100 is assembled (e.g., housing 120 may rest atop circuit board 170 in various examples). Processing module 160 may be electrically connected with circuit board 170 through appropriate electrical connections. As a result, infrared sensor assembly 128 may be electrically connected with processing module 160 through, for example, conductive electrical paths provided by: bond pads 142, complementary connections on inside surfaces of housing 120, electrical connections 126 of housing 120, connections 124, and circuit board 170. Advantageously, such an arrangement may be implemented without requiring wire bonds to be provided between infrared sensor assembly 128 and processing module 160.

In various examples, electrical connections 126 in housing 120 may be made from any desired material (e.g., copper or any other appropriate conductive material). In one example, electrical connections 126 may aid in dissipating heat from infrared imaging module 100.

Other connections may be used in other examples. For example, sensor assembly 128 may be attached to processing module 160 through a ceramic board that connects to sensor assembly 128 by wire bonds and to processing module 160 by a ball grid array (BGA). In another example, sensor assembly 128 may be mounted directly on a rigid flexible board and electrically connected with wire bonds, and processing module 160 may be mounted and connected to the rigid flexible board with wire bonds or a BGA.

The various implementations of infrared imaging module 100 and host device 102 set forth herein are provided for purposes of example, rather than limitation. In this regard, any of the various techniques described herein may be applied to any infrared camera system, infrared imager, or other device for performing infrared/thermal imaging.

Substrate 140 of infrared sensor assembly 128 may be mounted on base 150. In various examples, base 150 (e.g., a pedestal) may be made, for example, of copper formed by metal injection molding (MIM) and provided with a black oxide or nickel-coated finish. In various examples, base 150 may be made of any desired material, such as for example zinc, aluminum, or magnesium, as desired for a given application and may be formed by any desired applicable process, such as for example aluminum casting, MIM, or zinc rapid casting, as may be desired for particular applications. In various examples, base 150 may be implemented to provide structural support, various circuit paths, thermal heat sink properties, and other features where appropriate. In one example, base 150 may be a multi-layer structure implemented at least in part using ceramic material.

In various examples, circuit board 170 may receive housing 120 and thus may physically support the various components of infrared imaging module 100. In various examples, circuit board 170 may be implemented as a printed circuit board (e.g., an FR4 circuit board or other types of circuit boards), a rigid or flexible interconnect (e.g., tape or other type of interconnects), a flexible circuit substrate, a flexible plastic substrate, or other appropriate structures. In various examples, base 150 may be implemented with the various features and attributes described for circuit board 170, and vice versa.

Socket 104 may include a cavity 106 configured to receive infrared imaging module 100 (e.g., as shown in the assembled view of Fig. 2). Infrared imaging module 100 and/or socket 104 may include appropriate tabs, arms, pins, fasteners, or any other appropriate engagement members which may be used to secure infrared imaging module 100 to or within socket 104 using friction, tension, adhesion, and/or any other appropriate manner. Socket 104 may include engagement members 107 that may engage surfaces 109 of housing 120 when infrared imaging module 100 is inserted into a cavity 106 of socket 104. Other types of engagement members may be used in other examples.

Infrared imaging module 100 may be electrically connected with socket 104 through appropriate electrical connections (e.g., contacts, pins, wires, or any other appropriate connections). For example, socket 104 may include electrical connections 108 which may contact corresponding electrical connections of infrared imaging module 100 (e.g., interconnect pads, contacts, or other electrical connections on side or bottom surfaces of circuit board 170, bond pads 142 or other electrical connections on base 150, or other connections). Electrical connections 108 may be made from any desired material (e.g., copper or any other appropriate conductive material). In one example, electrical connections 108 may be mechanically biased to press against electrical connections of infrared imaging module 100 when infrared imaging module 100 is inserted into cavity 106 of socket 104. In one example, electrical connections 108 may at least partially secure infrared imaging module 100 in socket 104. Other types of electrical connections may be used in other examples.

Socket 104 may be electrically connected with host device 102 through similar types of electrical connections. For example, in one example, host device 102 may include electrical connections (e.g., soldered connections, snap-in connections, or other connections) that connect with electrical connections 108 passing through apertures 190. In various examples, such electrical connections may be made to the sides and/or bottom of socket 104.

Various components of infrared imaging module 100 may be implemented with flip chip technology which may be used to mount components directly to circuit boards without the additional clearances typically needed for wire bond connections. Flip chip connections may be used, as an example, to reduce the overall size of infrared imaging module 100 for use in compact small form factor applications. For example, processing module 160 may be mounted to circuit board 170 using flip chip connections. For example, infrared imaging module 100 may be implemented with such flip chip configurations.

In various examples, infrared imaging module 100 and/or associated components may be implemented in accordance with various techniques (e.g., wafer level packaging techniques) as set forth in U.S. Patent Application No. 12/844,124 filed July 27, 2010, and U.S. Provisional Patent Application No. 61/469,651 filed March 30, 2011. Furthermore, in accordance with one or more examples, infrared imaging module 100 and/or associated components may be implemented, calibrated, tested, and/or used in accordance with various techniques, such as for example as set forth in U.S. Patent No. 7,470,902 issued December 30, 2008, U.S. Patent No. 6,028,309 issued February 22, 2000, U.S. Patent No. 6,812,465 issued November 2, 2004, U.S. Patent No. 7,034,301 issued April 25, 2006, U.S. Patent No. 7,679,048 issued March 16, 2010, U.S. Patent No. 7,470,904 issued December 30, 2008, U.S. Patent Application No. 12/202,880 filed September 2, 2008, and U.S. Patent Application No. 12/202,896 filed September 2, 2008.

Referring again to Fig. 1, in various examples, host device 102 may include shutter 105. In this regard, shutter 105 may be selectively positioned over socket 104 (e.g., as identified by arrows 103) while infrared imaging module 100 is installed therein. In this regard, shutter 105 may be used, for example, to protect infrared imaging module 100 when not in use. Shutter 105 may also be used as a temperature reference as part of a calibration process (e.g., a NUC process or other calibration processes) for infrared imaging module 100 as would be understood by one skilled in the art.

In various examples, shutter 105 may be made from various materials such as, for example, polymers, glass, aluminum (e.g., painted or anodized) or other materials. In various embodiments, shutter 105 may include one or more coatings to selectively filter electromagnetic radiation and/or adjust various optical properties of shutter 105 (e.g., a uniform blackbody coating or a reflective gold coating).

In another example, shutter 105 may be fixed in place to protect infrared imaging module 100 at all times. In this case, shutter 105 or a portion of shutter 105 may be made from appropriate materials (e.g., polymers or infrared transmitting materials such as silicon, germanium, zinc selenide, or chalcogenide glasses) that do not substantially filter desired infrared wavelengths. In another embodiment, a shutter may be implemented as part of infrared imaging module 100 (e.g., within or as part of a lens barrel or other components of infrared imaging module 100), as would be understood by one skilled in the art.

Alternatively, in another example, a shutter (e.g., shutter 105 or other type of external or internal shutter) need not be provided, but rather a NUC process or other type of calibration may be performed using shutterless techniques. In another example, a NUC process or other type of calibration using shutterless techniques may be performed in combination with shutter-based techniques.

Infrared imaging module 100 and host device 102 may be implemented in accordance with any of the various techniques set forth in U.S. Provisional Patent Application No. 61/495,873 filed June 10, 2011, U.S. Provisional Patent Application No. 61/495,879 filed June 10, 2011, and U.S. Provisional Patent Application No. 61/495,888 filed June 10, 2011.

In various examples, the components of host device 102 and/or infrared imaging module 100 may be implemented as a local or distributed system with components in communication with each other over wired and/or wireless networks. Accordingly, the various operations identified in this disclosure may be performed by local and/or remote components as may be desired in particular implementations.

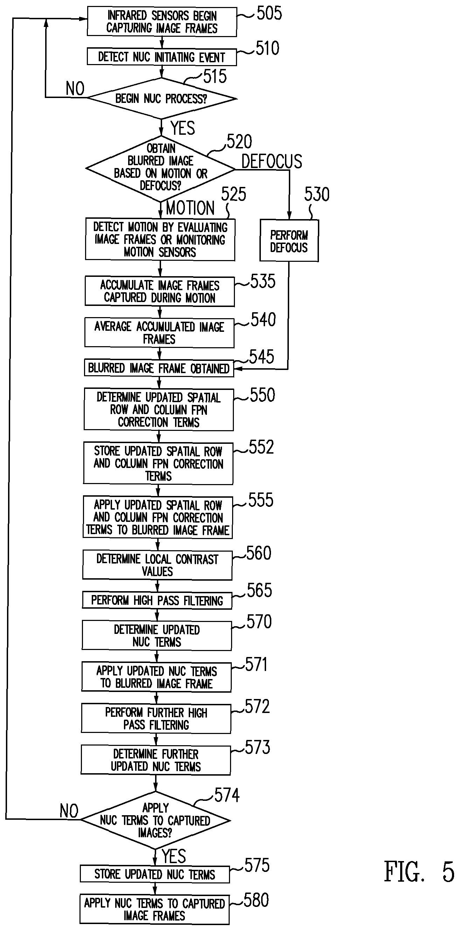

Fig. 5 illustrates a flow diagram of various operations to determine NUC terms in accordance with an example of the disclosure. In some examples, the operations of Fig. 5 may be performed by processing module 160 or processor 195 (both also generally referred to as a processor) operating on image frames captured by infrared sensors 132.

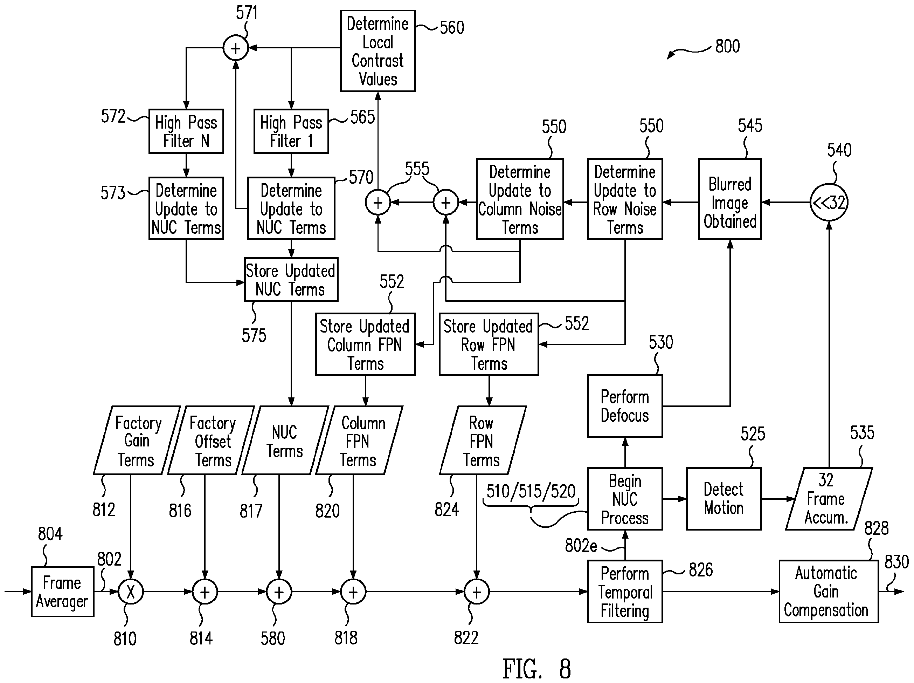

In block 505, infrared sensors 132 begin capturing image frames of a scene. Typically, the scene will be the real world environment in which host device 102 is currently located. In this regard, shutter 105 (if optionally provided) may be opened to permit infrared imaging module to receive infrared radiation from the scene. Infrared sensors 132 may continue capturing image frames during all operations shown in Fig. 5. In this regard, the continuously captured image frames may be used for various operations as further discussed. In one example, the captured image frames may be temporally filtered (e.g., in accordance with the process of block 826 further described herein with regard to Fig. 8) and be processed by other terms (e.g., factory gain terms 812, factory offset terms 816, previously determined NUC terms 817, column FPN terms 820, and row FPN terms 824 as further described herein with regard to Fig. 8) before they are used in the operations shown in Fig. 5.

In block 510, a NUC process initiating event is detected. In one example, the NUC process may be initiated in response to physical movement of host device 102. Such movement may be detected, for example, by motion sensors 194 which may be polled by a processor. In one example, a user may move host device 102 in a particular manner, such as by intentionally waving host device 102 back and forth in an "erase" or "swipe" movement. In this regard, the user may move host device 102 in accordance with a predetermined speed and direction (velocity), such as in an up and down, side to side, or other pattern to initiate the NUC process. In this example, the use of such movements may permit the user to intuitively operate host device 102 to simulate the "erasing" of noise in captured image frames.

In another example, a NUC process may be initiated by host device 102 if motion exceeding a threshold value is detected (e.g., motion greater than expected for ordinary use). It is contemplated that any desired type of spatial translation of host device 102 may be used to initiate the NUC process.

In yet another example, a NUC process may be initiated by host device 102 if a minimum time has elapsed since a previously performed NUC process. In a further example, a NUC process may be initiated by host device 102 if infrared imaging module 100 has experienced a minimum temperature change since a previously performed NUC process. In a still further example, a NUC process may be continuously initiated and repeated.

In block 515, after a NUC process initiating event is detected, it is determined whether the NUC process should actually be performed. In this regard, the NUC process may be selectively initiated based on whether one or more additional conditions are met. For example, the NUC process may not be performed unless a minimum time has elapsed since a previously performed NUC process. In another example, the NUC process may not be performed unless infrared imaging module 100 has experienced a minimum temperature change since a previously performed NUC process. Other criteria or conditions may be used in other examples. If appropriate criteria or conditions have been met, then the flow diagram continues to block 520. Otherwise, the flow diagram returns to block 505.

In the NUC process, blurred image frames may be used to determine NUC terms which may be applied to captured image frames to correct for FPN. As discussed, in one example, the blurred image frames may be obtained by accumulating multiple image frames of a moving scene (e.g., captured while the scene and/or the thermal imager is in motion). In another example, the blurred image frames may be obtained by defocusing an optical element or other component of the thermal imager.

Accordingly, in block 520 a choice of either approach is provided. If the motion-based approach is used, then the flow diagram continues to block 525. If the defocus-based approach is used, then the flow diagram continues to block 530.

Referring now to the motion-based approach, in block 525 motion is detected. For example, in one example, motion may be detected based on the image frames captured by infrared sensors 132. In this regard, an appropriate motion detection process (e.g., an image registration process, a frame-to-frame difference calculation, or other appropriate process) may be applied to captured image frames to determine whether motion is present (e.g., whether static or moving image frames have been captured). For example, it can be determined whether pixels or regions around the pixels of consecutive image frames have changed more than a user defined amount (e.g., a percentage and/or threshold value). If at least a given percentage of pixels have changed by at least the user defined amount, then motion will be detected with sufficient certainty to proceed to block 535.

In another example, motion may be determined on a per pixel basis, wherein only pixels that exhibit significant changes are accumulated to provide the blurred image frame. For example, counters may be provided for each pixel and used to ensure that the same number of pixel values are accumulated for each pixel, or used to average the pixel values based on the number of pixel values actually accumulated for each pixel. Other types of image-based motion detection may be performed such as performing a Radon transform.

In another example, motion may be detected based on data provided by motion sensors 194. In one example, such motion detection may include detecting whether host device 102 is moving along a relatively straight trajectory through space. For example, if host device 102 is moving along a relatively straight trajectory, then it is possible that certain objects appearing in the imaged scene may not be sufficiently blurred (e.g., objects in the scene that may be aligned with or moving substantially parallel to the straight trajectory). Thus, in such an example, the motion detected by motion sensors 194 may be conditioned on host device 102 exhibiting, or not exhibiting, particular trajectories.

In yet another example, both a motion detection process and motion sensors 194 may be used. Thus, using any of these various examples, a determination can be made as to whether or not each image frame was captured while at least a portion of the scene and host device 102 were in motion relative to each other (e.g., which may be caused by host device 102 moving relative to the scene, at least a portion of the scene moving relative to host device 102, or both).

It is expected that the image frames for which motion was detected may exhibit some secondary blurring of the captured scene (e.g., blurred thermal image data associated with the scene) due to the thermal time constants of infrared sensors 132 (e.g., microbolometer thermal time constants) interacting with the scene movement.

In block 535, image frames for which motion was detected are accumulated. For example, if motion is detected for a continuous series of image frames, then the image frames of the series may be accumulated. As another example, if motion is detected for only some image frames, then the non-moving image frames may be skipped and not included in the accumulation. Thus, a continuous or discontinuous set of image frames may be selected to be accumulated based on the detected motion.

In block 540, the accumulated image frames are averaged to provide a blurred image frame. Because the accumulated image frames were captured during motion, it is expected that actual scene information will vary between the image frames and thus cause the scene information to be further blurred in the resulting blurred image frame (block 545).

In contrast, FPN (e.g., caused by one or more components of infrared imaging module 100) will remain fixed over at least short periods of time and over at least limited changes in scene irradiance during motion. As a result, image frames captured in close proximity in time and space during motion will suffer from identical or at least very similar FPN. Thus, although scene information may change in consecutive image frames, the FPN will stay essentially constant. By averaging, multiple image frames captured during motion will blur the scene information, but will not blur the FPN. As a result, FPN will remain more clearly defined in the blurred image frame provided in block 545 than the scene information.

In one example, 32 or more image frames are accumulated and averaged in blocks 535 and 540. However, any desired number of image frames may be used in other examples, but with generally decreasing correction accuracy as frame count is decreased.

Referring now to the defocus-based approach, in block 530, a defocus operation may be performed to intentionally defocus the image frames captured by infrared sensors 132. For example, one or more actuators 199 may be used to adjust, move, or otherwise translate optical element 180, infrared sensor assembly 128, and/or other components of infrared imaging module 100 to cause infrared sensors 132 to capture a blurred (e.g., unfocused) image frame of the scene. Other non-actuator based techniques are also contemplated for intentionally defocusing infrared image frames such as, for example, manual (e.g., user-initiated) defocusing.

Although the scene may appear blurred in the image frame, FPN (e.g., caused by one or more components of infrared imaging module 100) will remain unaffected by the defocusing operation. As a result, a blurred image frame of the scene will be provided (block 545) with FPN remaining more clearly defined in the blurred image than the scene information.

In the above discussion, the defocus-based approach has been described with regard to a single captured image frame. In another example, the defocus-based approach may include accumulating multiple image frames while the infrared imaging module 100 has been defocused and averaging the defocused image frames to remove the effects of temporal noise and provide a blurred image frame in block 545.

Thus, it will be appreciated that a blurred image frame may be provided in block 545 by either the motion-based approach or the defocus-based approach. Because much of the scene information will be blurred by either motion, defocusing, or both, the blurred image frame may be effectively considered a low pass filtered version of the original captured image frames with respect to scene information.

In block 550, the blurred image frame is processed to determine updated row and column FPN terms (e.g., if row and column FPN terms have not been previously determined then the updated row and column FPN terms may be new row and column FPN terms in the first iteration of block 550). As used in this disclosure, the terms row and column may be used interchangeably depending on the orientation of infrared sensors 132 and/or other components of infrared imaging module 100.

In one example, block 550 includes determining a spatial FPN correction term for each row of the blurred image frame (e.g., each row may have its own spatial FPN correction term), and also determining a spatial FPN correction term for each column of the blurred image frame (e.g., each column may have its own spatial FPN correction term). Such processing may be used to reduce the spatial and slowly varying (1/f) row and column FPN inherent in thermal imagers caused by, for example, 1/f noise characteristics of amplifiers in ROIC 402 which may manifest as vertical and horizontal stripes in image frames.

Advantageously, by determining spatial row and column FPN terms using the blurred image frame, there will be a reduced risk of vertical and horizontal objects in the actual imaged scene from being mistaken for row and column noise (e.g., real scene content will be blurred while FPN remains unblurred).

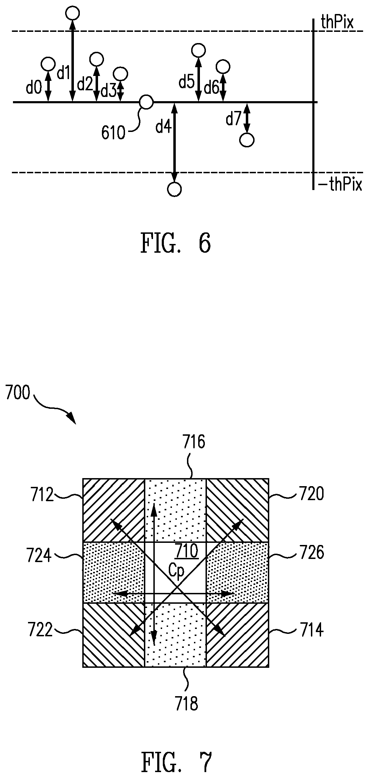

In one example, row and column FPN terms may be determined by considering differences between neighboring pixels of the blurred image frame. For example, Fig. 6 illustrates differences between neighboring pixels in accordance with an example of the disclosure. Specifically, in Fig. 6 a pixel 610 is compared to its 8 nearest horizontal neighbors: d0-d3 on one side and d4-d7 on the other side. Differences between the neighbor pixels can be averaged to obtain an estimate of the offset error of the illustrated group of pixels. An offset error may be calculated for each pixel in a row or column and the average result may be used to correct the entire row or column.

To prevent real scene data from being interpreted as noise, upper and lower threshold values may be used (thPix and -thPix). Pixel values falling outside these threshold values (pixels d1 and d4 in this example) are not used to obtain the offset error. In addition, the maximum amount of row and column FPN correction may be limited by these threshold values.

Further techniques for performing spatial row and column FPN correction processing are set forth in U.S. Patent Application No. 12/396,340 filed March 2, 2009.

Referring again to Fig. 5, the updated row and column FPN terms determined in block 550 are stored (block 552) and applied (block 555) to the blurred image frame provided in block 545. After these terms are applied, some of the spatial row and column FPN in the blurred image frame may be reduced. However, because such terms are applied generally to rows and columns, additional FPN may remain such as spatially uncorrelated FPN associated with pixel to pixel drift or other causes. Neighborhoods of spatially correlated FPN may also remain which may not be directly associated with individual rows and columns. Accordingly, further processing may be performed as discussed below to determine NUC terms.

In block 560, local contrast values (e.g., edges or absolute values of gradients between adjacent or small groups of pixels) in the blurred image frame are determined. If scene information in the blurred image frame includes contrasting areas that have not been significantly blurred (e.g., high contrast edges in the original scene data), then such features may be identified by a contrast determination process in block 560.

For example, local contrast values in the blurred image frame may be calculated, or any other desired type of edge detection process may be applied to identify certain pixels in the blurred image as being part of an area of local contrast. Pixels that are marked in this manner may be considered as containing excessive high spatial frequency scene information that would be interpreted as FPN (e.g., such regions may correspond to portions of the scene that have not been sufficiently blurred). As such, these pixels may be excluded from being used in the further determination of NUC terms. In one embodiment, such contrast detection processing may rely on a threshold that is higher than the expected contrast value associated with FPN (e.g., pixels exhibiting a contrast value higher than the threshold may be considered to be scene information, and those lower than the threshold may be considered to be exhibiting FPN).

In one example, the contrast determination of block 560 may be performed on the blurred image frame after row and column FPN terms have been applied to the blurred image frame (e.g., as shown in Fig. 5). In another example, block 560 may be performed prior to block 550 to determine contrast before row and column FPN terms are determined (e.g., to prevent scene based contrast from contributing to the determination of such terms).

Following block 560, it is expected that any high spatial frequency content remaining in the blurred image frame may be generally attributed to spatially uncorrelated FPN. In this regard, following block 560, much of the other noise or actual desired scene based information has been removed or excluded from the blurred image frame due to: intentional blurring of the image frame (e.g., by motion or defocusing in blocks 520 through 545), application of row and column FPN terms (block 555), and contrast determination (block 560).

Thus, it can be expected that following block 560, any remaining high spatial frequency content (e.g., exhibited as areas of contrast or differences in the blurred image frame) may be attributed to spatially uncorrelated FPN. Accordingly, in block 565, the blurred image frame is high pass filtered. In one example, this may include applying a high pass filter to extract the high spatial frequency content from the blurred image frame. In another example, this may include applying a low pass filter to the blurred image frame and taking a difference between the low pass filtered image frame and the unfiltered blurred image frame to obtain the high spatial frequency content. In accordance with various examples of the present disclosure, a high pass filter may be implemented by calculating a mean difference between a sensor signal (e.g., a pixel value) and its neighbors.

In block 570, a flat field correction process is performed on the high pass filtered blurred image frame to determine updated NUC terms (e.g., if a NUC process has not previously been performed then the updated NUC terms may be new NUC terms in the first iteration of block 570).

For example, Fig. 7 illustrates a flat field correction technique 700 in accordance with an example of the disclosure. In Fig. 7, a NUC term may be determined for each pixel 710 of the blurred image frame using the values of its neighboring pixels 712 to 726. For each pixel 710, several gradients may be determined based on the absolute difference between the values of various adjacent pixels. For example, absolute value differences may be determined between: pixels 712 and 714 (a left to right diagonal gradient), pixels 716 and 718 (a top to bottom vertical gradient), pixels 720 and 722 (a right to left diagonal gradient), and pixels 724 and 726 (a left to right horizontal gradient).

These absolute differences may be summed to provide a summed gradient for pixel 710. A weight value may be determined for pixel 710 that is inversely proportional to the summed gradient. This process may be performed for all pixels 710 of the blurred image frame until a weight value is provided for each pixel 710. For areas with low gradients (e.g., areas that are blurry or have low contrast), the weight value will be close to one. Conversely, for areas with high gradients, the weight value will be zero or close to zero. The update to the NUC term as estimated by the high pass filter is multiplied with the weight value.

In one example, the risk of introducing scene information into the NUC terms can be further reduced by applying some amount of temporal damping to the NUC term determination process. For example, a temporal damping factor λ between 0 and 1 may be chosen such that the new NUC term (NUC_{NEW}) stored is a weighted average of the old NUC term (NUC_{OLD}) and the estimated updated NUC term (NUC_{UPDATE}). In one example, this can be expressed as NUC_{NEW} = λ·NUC_{OLD}+(1-λ)·(NUC_{OLD}+NUC_{UPDATE}).

Although the determination of NUC terms has been described with regard to gradients, local contrast values may be used instead where appropriate. Other techniques may also be used such as, for example, standard deviation calculations. Other types flat field correction processes may be performed to determine NUC terms including, for example, various processes identified in U.S. Patent No. 6,028,309 issued February 22, 2000, U.S. Patent No. 6,812,465 issued November 2, 2004, and U.S. Patent Application No. 12/114,865 filed May 5, 2008.

Referring again to Fig. 5, block 570 may include additional processing of the NUC terms. For example, to preserve the scene signal mean, the sum of all NUC terms may be normalized to zero by subtracting the NUC term mean from each NUC term. Also in block 570, to avoid row and column noise from affecting the NUC terms, the mean value of each row and column may be subtracted from the NUC terms for each row and column. As a result, row and column FPN filters using the row and column FPN terms determined in block 550 may be better able to filter out row and column noise in further iterations (e.g., as further shown in Fig. 8) after the NUC terms are applied to captured images (e.g., in block 580 further discussed herein). In this regard, the row and column FPN filters may in general use more data to calculate the per row and per column offset coefficients (e.g., row and column FPN terms) and may thus provide a more robust alternative for reducing spatially correlated FPN than the NUC terms which are based on high pass filtering to capture spatially uncorrelated noise.

In blocks 571-573, additional high pass filtering and further determinations of updated NUC terms may be optionally performed to remove spatially correlated FPN with lower spatial frequency than previously removed by row and column FPN terms. In this regard, some variability in infrared sensors 132 or other components of infrared imaging module 100 may result in spatially correlated FPN noise that cannot be easily modeled as row or column noise. Such spatially correlated FPN may include, for example, window defects on a sensor package or a cluster of infrared sensors 132 that respond differently to irradiance than neighboring infrared sensors 132. In one example, such spatially correlated FPN may be mitigated with an offset correction. If the amount of such spatially correlated FPN is significant, then the noise may also be detectable in the blurred image frame. Since this type of noise may affect a neighborhood of pixels, a high pass filter with a small kernel may not detect the FPN in the neighborhood (e.g., all values used in high pass filter may be taken from the neighborhood of affected pixels and thus may be affected by the same offset error). For example, if the high pass filtering of block 565 is performed with a small kernel (e.g., considering only immediately adjacent pixels that fall within a neighborhood of pixels affected by spatially correlated FPN), then broadly distributed spatially correlated FPN may not be detected.

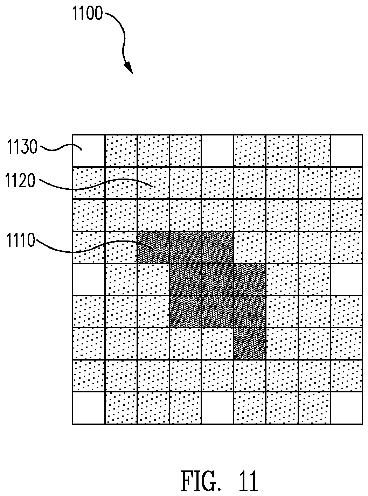

For example, Fig. 11 illustrates spatially correlated FPN in a neighborhood of pixels in accordance with an example of the disclosure. As shown in a sample image frame 1100, a neighborhood of pixels 1110 may exhibit spatially correlated FPN that is not precisely correlated to individual rows and columns and is distributed over a neighborhood of several pixels (e.g., a neighborhood of approximately 4 by 4 pixels in this example). Sample image frame 1100 also includes a set of pixels 1120 exhibiting substantially uniform response that are not used in filtering calculations, and a set of pixels 1130 that are used to estimate a low pass value for the neighborhood of pixels 1110. In one example, pixels 1130 may be a number of pixels divisible by two in order to facilitate efficient hardware or software calculations.

Referring again to Fig. 5, in blocks 571-573, additional high pass filtering and further determinations of updated NUC terms may be optionally performed to remove spatially correlated FPN such as exhibited by pixels 1110. In block 571, the updated NUC terms determined in block 570 are applied to the blurred image frame. Thus, at this time, the blurred image frame will have been initially corrected for spatially correlated FPN (e.g., by application of the updated row and column FPN terms in block 555), and also initially corrected for spatially uncorrelated FPN (e.g., by application of the updated NUC terms applied in block 571).

In block 572, a further high pass filter is applied with a larger kernel than was used in block 565, and further updated NUC terms may be determined in block 573. For example, to detect the spatially correlated FPN present in pixels 1110, the high pass filter applied in block 572 may include data from a sufficiently large enough neighborhood of pixels such that differences can be determined between unaffected pixels (e.g., pixels 1120) and affected pixels (e.g., pixels 1110). For example, a low pass filter with a large kernel can be used (e.g., an N by N kernel that is much greater than 3 by 3 pixels) and the results may be subtracted to perform appropriate high pass filtering.

In one example, for computational efficiency, a sparse kernel may be used such that only a small number of neighboring pixels inside an N by N neighborhood are used. For any given high pass filter operation using distant neighbors (e.g., a large kernel), there is a risk of modeling actual (potentially blurred) scene information as spatially correlated FPN. Accordingly, in one example, the temporal damping factor λ may be set close to 1 for updated NUC terms determined in block 573.

In various examples, blocks 571-573 may be repeated (e.g., cascaded) to iteratively perform high pass filtering with increasing kernel sizes to provide further updated NUC terms further correct for spatially correlated FPN of desired neighborhood sizes. In one example, the decision to perform such iterations may be determined by whether spatially correlated FPN has actually been removed by the updated NUC terms of the previous performance of blocks 571-573.

After blocks 571-573 are finished, a decision is made regarding whether to apply the updated NUC terms to captured image frames (block 574). For example, if an average of the absolute value of the NUC terms for the entire image frame is less than a minimum threshold value, or greater than a maximum threshold value, the NUC terms may be deemed spurious or unlikely to provide meaningful correction. Alternatively, thresholding criteria may be applied to individual pixels to determine which pixels receive updated NUC terms. In one example, the threshold values may correspond to differences between the newly calculated NUC terms and previously calculated NUC terms. In another example, the threshold values may be independent of previously calculated NUC terms. Other tests may be applied (e.g., spatial correlation tests) to determine whether the NUC terms should be applied.

If the NUC terms are deemed spurious or unlikely to provide meaningful correction, then the flow diagram returns to block 505. Otherwise, the newly determined NUC terms are stored (block 575) to replace previous NUC terms (e.g., determined by a previously performed iteration of Fig. 5) and applied (block 580) to captured image frames.

Fig. 8 illustrates various image processing techniques of Fig. 5 and other operations applied in an image processing pipeline 800 in accordance with an example of the disclosure. In this regard, pipeline 800 identifies various operations of Fig. 5 in the context of an overall iterative image processing scheme for correcting image frames provided by infrared imaging module 100. In some examples, pipeline 800 may be provided by processing module 160 or processor 195 (both also generally referred to as a processor) operating on image frames captured by infrared sensors 132.

Image frames captured by infrared sensors 132 may be provided to a frame averager 804 that integrates multiple image frames to provide image frames 802 with an improved signal to noise ratio. Frame averager 804 may be effectively provided by infrared sensors 132, ROIC 402, and other components of infrared sensor assembly 128 that are implemented to support high image capture rates. For example, in one example, infrared sensor assembly 128 may capture infrared image frames at a frame rate of 240 Hz (e.g., 240 images per second). In this example, such a high frame rate may be implemented, for example, by operating infrared sensor assembly 128 at relatively low voltages (e.g., compatible with mobile telephone voltages) and by using a relatively small array of infrared sensors 132 (e.g., an array of 64 by 64 infrared sensors in one example).

In one example, such infrared image frames may be provided from infrared sensor assembly 128 to processing module 160 at a high frame rate (e.g., 240 Hz or other frame rates). In another example, infrared sensor assembly 128 may integrate over longer time periods, or multiple time periods, to provide integrated (e.g., averaged) infrared image frames to processing module 160 at a lower frame rate (e.g., 30 Hz, 9 Hz, or other frame rates). Further information regarding implementations that may be used to provide high image capture rates may be found in U.S. Provisional Patent Application No. 61/495,879 previously referenced herein.

Image frames 802 proceed through pipeline 800 where they are adjusted by various terms, temporally filtered, used to determine the various adjustment terms, and gain compensated.

In blocks 810 and 814, factory gain terms 812 and factory offset terms 816 are applied to image frames 802 to compensate for gain and offset differences, respectively, between the various infrared sensors 132 and/or other components of infrared imaging module 100 determined during manufacturing and testing.

In block 580, NUC terms 817 are applied to image frames 802 to correct for FPN as discussed. In one example, if NUC terms 817 have not yet been determined (e.g., before a NUC process has been initiated), then block 580 may not be performed or initialization values may be used for NUC terms 817 that result in no alteration to the image data (e.g., offsets for every pixel would be equal to zero).

In blocks 818 and 822, column FPN terms 820 and row FPN terms 824, respectively, are applied to image frames 802. Column FPN terms 820 and row FPN terms 824 may be determined in accordance with block 550 as discussed. In one example, if the column FPN terms 820 and row FPN terms 824 have not yet been determined (e.g., before a NUC process has been initiated), then blocks 818 and 822 may not be performed or initialization values may be used for the column FPN terms 820 and row FPN terms 824 that result in no alteration to the image data (e.g., offsets for every pixel would be equal to zero).

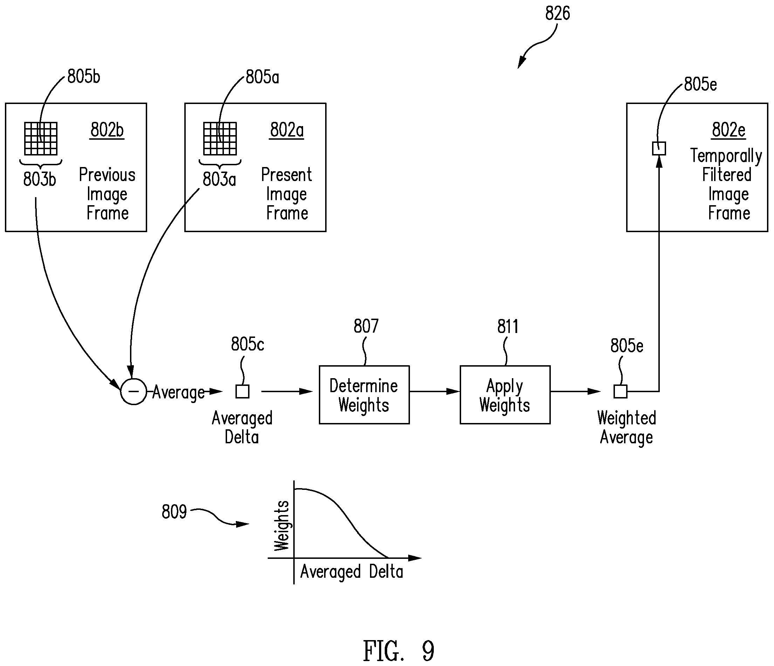

In block 826, temporal filtering is performed on image frames 802 in accordance with a temporal noise reduction (TNR) process. Fig. 9 illustrates a TNR process in accordance with an example of the disclosure. In Fig. 9, a presently received image frame 802a and a previously temporally filtered image frame 802b are processed to determine a new temporally filtered image frame 802e. Image frames 802a and 802b include local neighborhoods of pixels 803a and 803b centered around pixels 805a and 805b, respectively. Neighborhoods 803a and 803b correspond to the same locations within image frames 802a and 802b and are subsets of the total pixels in image frames 802a and 802b. In the illustrated example, neighborhoods 803a and 803b include areas of 5 by 5 pixels. Other neighborhood sizes may be used in other examples.

Differences between corresponding pixels of neighborhoods 803a and 803b are determined and averaged to provide an averaged delta value 805c for the location corresponding to pixels 805a and 805b. Averaged delta value 805c may be used to determine weight values in block 807 to be applied to pixels 805a and 805b of image frames 802a and 802b.

In one example, as shown in graph 809, the weight values determined in block 807 may be inversely proportional to averaged delta value 805c such that weight values drop rapidly towards zero when there are large differences between neighborhoods 803a and 803b. In this regard, large differences between neighborhoods 803a and 803b may indicate that changes have occurred within the scene (e.g., due to motion) and pixels 802a and 802b may be appropriately weighted, in one example, to avoid introducing blur across frame-to-frame scene changes. Other associations between weight values and averaged delta value 805c may be used in various embodiments.

The weight values determined in block 807 may be applied to pixels 805a and 805b to determine a value for corresponding pixel 805e of image frame 802e (block 811). In this regard, pixel 805e may have a value that is a weighted average (or other combination) of pixels 805a and 805b, depending on averaged delta value 805c and the weight values determined in block 807.

For example, pixel 805e of temporally filtered image frame 802e may be a weighted sum of pixels 805a and 805b of image frames 802a and 802b. If the average difference between pixels 805a and 805b is due to noise, then it may be expected that the average change between neighborhoods 805a and 805b will be close to zero (e.g., corresponding to the average of uncorrelated changes). Under such circumstances, it may be expected that the sum of the differences between neighborhoods 805a and 805b will be close to zero. In this case, pixel 805a of image frame 802a may both be appropriately weighted so as to contribute to the value of pixel 805e.

However, if the sum of such differences is not zero (e.g., even differing from zero by a small amount in one example), then the changes may be interpreted as being attributed to motion instead of noise. Thus, motion may be detected based on the average change exhibited by neighborhoods 805a and 805b. Under these circumstances, pixel 805a of image frame 802a may be weighted heavily, while pixel 805b of image frame 802b may be weighted lightly.

Other examples are also contemplated. For example, although averaged delta value 805c has been described as being determined based on neighborhoods 805a and 805b, in other examples averaged delta value 805c may be determined based on any desired criteria (e.g., based on individual pixels or other types of groups of sets of pixels).

In the above examples, image frame 802a has been described as a presently received image frame and image frame 802b has been described as a previously temporally filtered image frame. In another example, image frames 802a and 802b may be first and second image frames captured by infrared imaging module 100 that have not been temporally filtered.

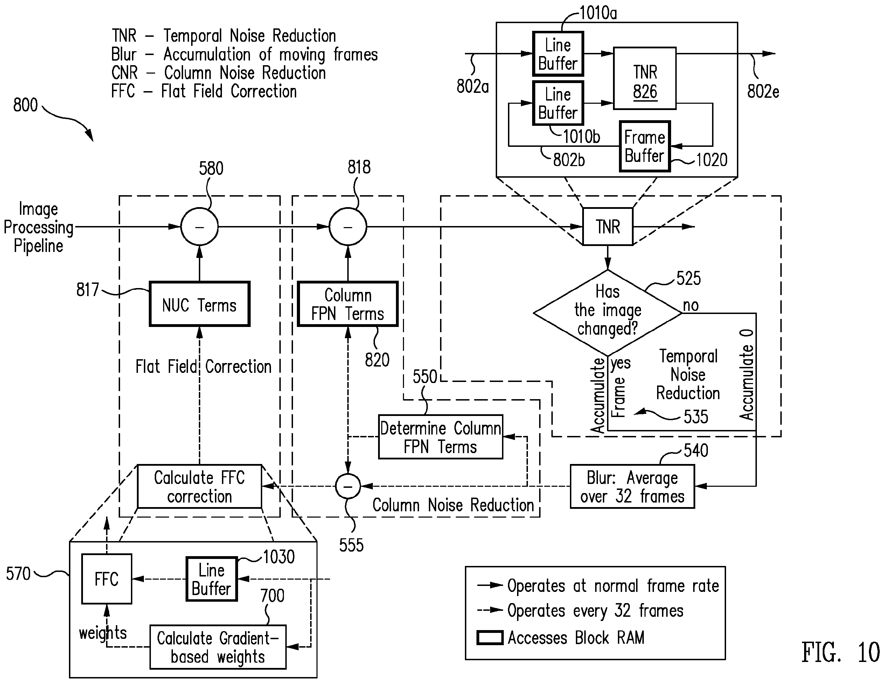

Fig. 10 illustrates further implementation details in relation to the TNR process of block 826. As shown in Fig. 10, image frames 802a and 802b may be read into line buffers 1010a and 1010b, respectively, and image frame 802b (e.g., the previous image frame) may be stored in a frame buffer 1020 before being read into line buffer 1010b. In one example, line buffers 1010a-b and frame buffer 1020 may be implemented by a block of random access memory (RAM) provided by any appropriate component of infrared imaging module 100 and/or host device 102.

Referring again to Fig. 8, image frame 802e may be passed to an automatic gain compensation block 828 for further processing to provide a result image frame 830 that may be used by host device 102 as desired.

Fig. 8 further illustrates various operations that may be performed to determine row and column FPN terms and NUC terms as discussed. In one example, these operations may use image frames 802e as shown in Fig. 8. Because image frames 802e have already been temporally filtered, at least some temporal noise may be removed and thus will not inadvertently affect the determination of row and column FPN terms 824 and 820 and NUC terms 817. In another example, non-temporally filtered image frames 802 may be used.

In Fig. 8, blocks 510, 515, and 520 of Fig. 5 are collectively represented together. As discussed, a NUC process may be selectively initiated and performed in response to various NUC process initiating events and based on various criteria or conditions. As also discussed, the NUC process may be performed in accordance with a motion-based approach (blocks 525, 535, and 540) or a defocus-based approach (block 530) to provide a blurred image frame (block 545). Fig. 8 further illustrates various additional blocks 550, 552, 555, 560, 565, 570, 571, 572, 573, and 575 previously discussed with regard to Fig. 5.

As shown in Fig. 8, row and column FPN terms 824 and 820 and NUC terms 817 may be determined and applied in an iterative fashion such that updated terms are determined using image frames 802 to which previous terms have already been applied. As a result, the overall process of Fig. 8 may repeatedly update and apply such terms to continuously reduce the noise in image frames 830 to be used by host device 102.

Referring again to Fig. 10, further implementation details are illustrated for various blocks of Figs. 5 and 8 in relation to pipeline 800. For example, blocks 525, 535, and 540 are shown as operating at the normal frame rate of image frames 802 received by pipeline 800. In the example shown in Fig. 10, the determination made in block 525 is represented as a decision diamond used to determine whether a given image frame 802 has sufficiently changed such that it may be considered an image frame that will enhance the blur if added to other image frames and is therefore accumulated (block 535 is represented by an arrow in this example) and averaged (block 540).

Also in Fig. 10, the determination of column FPN terms 820 (block 550) is shown as operating at an update rate that in this example is 1/32 of the sensor frame rate (e.g., normal frame rate) due to the averaging performed in block 540. Other update rates may be used in other embodiments. Although only column FPN terms 820 are identified in Fig. 10, row FPN terms 824 may be implemented in a similar fashion at the reduced frame rate.

Fig. 10 also illustrates further implementation details in relation to the NUC determination process of block 570. In this regard, the blurred image frame may be read to a line buffer 1030 (e.g., implemented by a block of RAM provided by any appropriate component of infrared imaging module 100 and/or host device 102). The flat field correction technique 700 of Fig. 7 may be performed on the blurred image frame.

In view of the present disclosure, it will be appreciated that techniques described herein may be used to remove various types of FPN (e.g., including very high amplitude FPN) such as spatially correlated row and column FPN and spatially uncorrelated FPN.

Other examples are also contemplated. For example, in one embodiment, the rate at which row and column FPN terms and/or NUC terms are updated can be inversely proportional to the estimated amount of blur in the blurred image frame and/or inversely proportional to the magnitude of local contrast values (e.g., determined in block 560).

In various examples, the described techniques may provide advantages over conventional shutter-based noise correction techniques. For example, by using a shutterless process, a shutter (e.g., such as shutter 105) need not be provided, thus permitting reductions in size, weight, cost, and mechanical complexity. Power and maximum voltage supplied to, or generated by, infrared imaging module 100 may also be reduced if a shutter does not need to be mechanically operated. Reliability will be improved by removing the shutter as a potential point of failure. A shutterless process also eliminates potential image interruption caused by the temporary blockage of the imaged scene by a shutter.

Also, by correcting for noise using intentionally blurred image frames captured from a real world scene (not a uniform scene provided by a shutter), noise correction may be performed on image frames that have irradiance levels similar to those of the actual scene desired to be imaged. This can improve the accuracy and effectiveness of noise correction terms determined in accordance with the various described techniques.

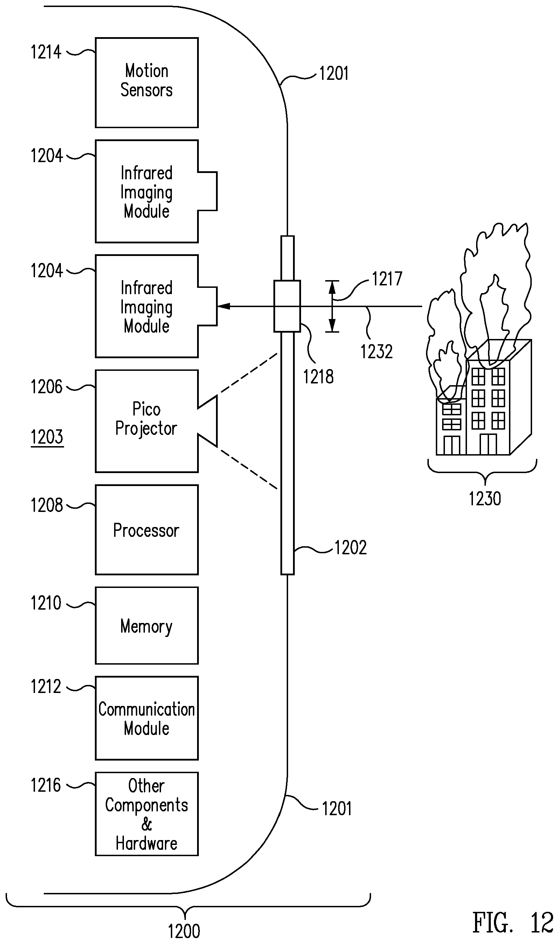

Referring now to Fig. 12, a block diagram is shown of a wearable apparatus 1200 in accordance with an embodiment of the disclosure. Wearable apparatus 1200 may include a shield 1202, one or more infrared imaging modules 1204, a projector 1206, a processor 1208, a memory 1210, a communication module 1212, motion sensors 1214, and other components and hardware 1216. In various embodiments, infrared imaging modules 1204, processor 1208, memory 1210, and motion sensors 1214 may be implemented in the same of similar manner as corresponding components of host device 102 of Fig. 1. Moreover, the various components of wearable apparatus 1200 may be configured to perform various NUC processes and other processes described herein.

Shield 1202 protects at least a portion of a user's face from an external environment 1230 when wearable apparatus 1200 is worn by a user (e.g., generally identified by reference number 1203 in Fig. 12). Shield 1202 may be made of polymers (e.g., polycarbonate), metal, or any other appropriate materials durable enough to provide a protective barrier against heat, intensive light rays, debris, and/or other harmful elements from external environment 1230. Shield 1202 may comprise or may be coated with one or more appropriate protective layers to enhance or provide protection against such harmful elements.

In various embodiments, shield 1202 may provide a protective barrier against external environment 1230 for various components of wearable apparatus 1200 as well. For example, infrared imaging modules 1204, projector 1206, processor 1208, memory 1210, communication module 1212, motion sensors 1214, and/or any appropriate components of wearable apparatus 1200 may be positioned internal to wearable apparatus 1200 (e.g., behind shield 1202 and away from external environment 1230), so that shield 1202 provides protection to these components in addition to protecting at least a portion of a user's face.

Although shield 1202 is illustrated in Fig. 12 as having a limited length, shield 1202 may be implemented with any desired size. Moreover, wearable apparatus 1200 may include one or more structural members 1201 to partially or completely enclose a face, head, or any desired portion of user 1203 (e.g., including the entirety of user 1203 if desired).

In one embodiment, shield 1202 may pass at least some visible light so that user 1203 can view external environment 1230 through shield 1202, while still being protected against harmful radiation (e.g., appropriate types of infrared radiation, ultraviolet radiation, and/or others), debris, and/or other elements. In another embodiment, a portion, a majority, or an entirety of shield 1202 may be opaque or nontransparent (e.g., when shield 1202 is made of metal). In some embodiments, a surface of shield 1202 may comprise a plate onto which images may be projected from projector 1206.

Infrared imaging modules 1204 may be small form factor infrared cameras or small form factor infrared imaging devices implemented in accordance with various embodiments disclosed herein. Infrared imaging modules 1204 may include an FPA implemented, for example, in accordance with various embodiments disclosed herein or others where appropriate.

Thus, unlike certain CCD-based or CMOS-based imaging sensors which may at best detect limited reflected short wave infrared (SWIR) rays (e.g., near infrared light) from illuminated objects, infrared imaging modules 1204 may be capable of detecting and capturing long wave infrared (LWIR) radiation, mid wave infrared (MWIR) radiation, and/or other radiation in thermal bands as may be desired. As such, infrared imaging modules 1204 may be configured to capture, process, and/or otherwise manage thermal images (e.g., images including thermal radiation data) of external environment 1230 even in complete darkness, and provide such images and data to processor 1208. For example, thermal images provided by infrared imaging modules 1204 may reveal invisible hazards such as gas leaks, thermal hot spots, or others. Such thermal images may include an accurate temperature reading of each pixel in the images. In this regard, it will be appreciated that thermal images captured and provided by infrared imaging modules 1204 are significantly more useful than images of amplified visible light and SWIR radiation that may be provided by conventional light intensifiers (e.g., night vision (NV) devices).

In one embodiment, wearable apparatus 1200 may comprise a plurality of infrared imaging modules 1204 to capture stereoscopic thermal images of external environment 1230. In another embodiment, one or more of a plurality of infrared imaging modules 1204 may provide fault tolerance by serving as backups to each other.

In various examples, infrared imaging modules 1204 and/or processor 1208 may be configured to provide automatic exposure control (e.g., by controlling signal gain, camera aperture, and/or shutter speed) to adjust to changes in the infrared intensity and temperature level of the external environment.

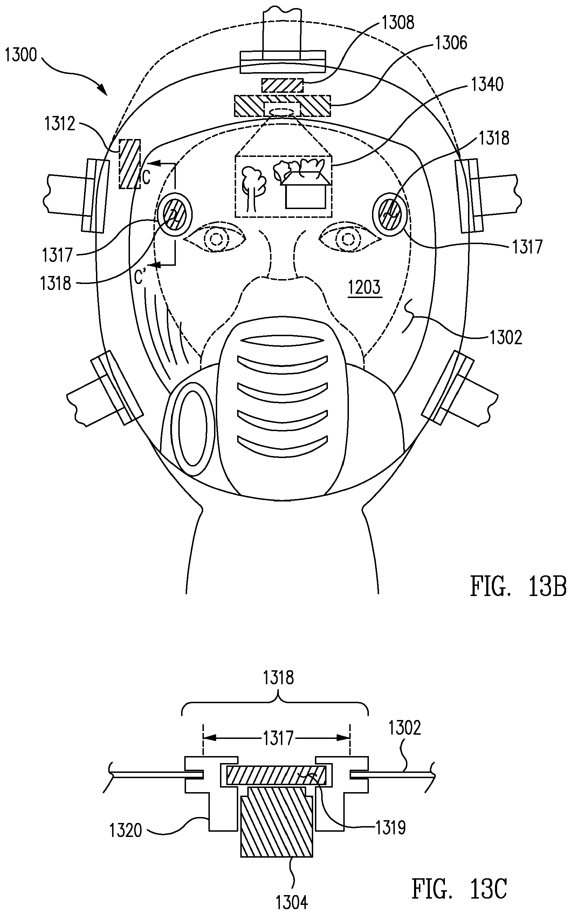

In various embodiments, one or more infrared imaging modules 1204 may be positioned behind shield 1202, so as to be protected from external environment 1230 by shield 1202. In such embodiments, shield 1202 may include an aperture 1217 sealed with a window assembly 1218 capable of passing infrared rays 1232 through to infrared imaging module 1204. Window assembly 1218 may be made of a material (e.g., silicon or other material) having a high transmittance for infrared light, so that infrared light emitted from external environment 1230 reaches infrared imaging module 1204 while shield 1202 blocks infrared light from user 1203 and/or various components of wearable apparatus 1200. In some embodiments, window assembly 1218 may be doped with appropriate material so that only infrared light in a desired wavelength range may pass through. Window assembly 1218 may be implemented in accordance with various types of structures as further described herein with regard to Fig. 13C.

Processor 1208 may be implemented as any appropriate processing device as described with regard to processor 195 in Fig. 1. In some embodiments, at least some part of processor 1208 may be implemented as part of infrared imaging modules 1204 and/or projector 1206.

Processor 1208 may be configured to receive one or more thermal images captured by infrared imaging module 1204, and to process the thermal images to generate user-viewable thermal images (e.g., thermograms) of external environment 1230. In one example, processor 1208 may generate and overlay information and/or alarms (e.g., a temperature reading, a gas detection alarm, mask pressure reading and alarm, oxygen tank reading and alarm, and/or others) onto user-viewable images. In some embodiments, processor 1208 may be configured to receive one or more thermal images from two or more infrared imaging modules 1204, and to appropriately combine the thermal images to generate stereoscopic user-viewable images (e.g., three dimensional thermograms) of external environment 1230 therefrom.

Projector 1206 may be implemented with any appropriate small form factor projector, including those known as "pico" or "micro" projectors. For example, projector 1206 may be sized small enough to be embedded inside personal electronic devices such as mobile phones or digital cameras. In various examples, projector 1206 may be implemented in accordance with various technologies such as digital light processing (DLP), liquid crystal on silicon (LCoS), laser beam steering (LBS), holographic laser projection (HLP), and/or others as appropriate.

Projector 1206 may be positioned so as to selectively project user-viewable thermal images onto an inner surface of shield 1202. For example, projector 1206 can be turned on to project user-viewable thermal images onto a portion of an inner surface of shield 1202 that is comfortably within a line of sight of user 1203 while wearable apparatus 1200 is worn, and can be turned off when user 1203 desires a clear view through shield 1202. Moreover, the direction of a beam from projector 1206 may be adjustable to project user-viewable thermal images onto an area of shield 1202 that is comfortable for viewing by a user, for example, for viewing images projected on shield 1202, and simultaneously viewing external environment 1230 through shield 1202. In contrast, conventional fixed-type displays, such as fixed LCD screens (e.g., viewed directly, through a scope or an objective lens), may obstruct a user's view even when not used, and may make it difficult for a user to adjust the screen's position or to simultaneously view visible light from external environment 1230 in realtime.

In various examples, projector 1206 may be configured to perform a distortion correction of user-viewable images projected on a surface of shield 1202, so that the user-viewable images appear flat and geometrically correct even when projected at an oblique angle and/or onto a curved surface of shield 1202. In some examples, projector 1206 may optically correct distortion using optical elements such as lenses, prisms, and mirrors.

In examples in which projector 1206 is implemented with HLP technology, distortion may be corrected through appropriate operations performed by projector 1206 and/or processor 1208. In this regard, such HLP technology may implement holographic processes to generate interference or diffraction patterns of an image instead of the image itself, and focused laser beams may be projected through such interference patterns to direct light as desired without relying on optical elements.

In some examples, projector 1206 may be configured to project two or more beams of light to present stereoscopic user-viewable images of external environment 1230 as described above. It is also contemplated that projector 1206 may be configured to project three dimensional user-viewable images using HLP technology.

Communication module 1212 may be configured to handle internal communication between various components of wearable apparatus 1200. For example, components such as infrared imaging modules 1204, projector 1206, and other sensors may transmit and receive data to and from processor 1208 through communication module 1212, which may manage wired and/or wireless connections (e.g., through proprietary RF links and/or through standard wireless communication protocols such as IEEE 802.11 WiFi standards and Bluetooth™) between the various components.

In some examples, communication module 1212 may be further configured to handle communication with devices external to wearable apparatus 1200. For example, communication module 1212 may transmit and receive user-viewable images generated by processor 1208 to and from other wearable apparatuses or a monitoring station so that user-viewable images can be shared with other users. In another example, communication module 1212 may handle a more conventional communication such as radio communication between users of wearable apparatus 1200.

Other components and hardware 1216 may be used to implement any features of wearable apparatus 1200 as may be desired for various applications. For example, other components may include various sensors, a microphone and speaker for voice communication, timers, a flashlight, and a visible light camera. Other hardware may include a mask frame, a hood, straps, fasteners, harnesses, connectors, hoses, and other various hardware and protective equipment and clothing as may be desired for certain applications of wearable apparatus 1200.

Thus, it will be appreciated that wearable apparatus 1200 may be implemented as any type of wearable device, equipment, gear, mask, helmet, garment, and/or clothing that includes shield 1202 to protect at least a portion of a user's face from external environment 1230.

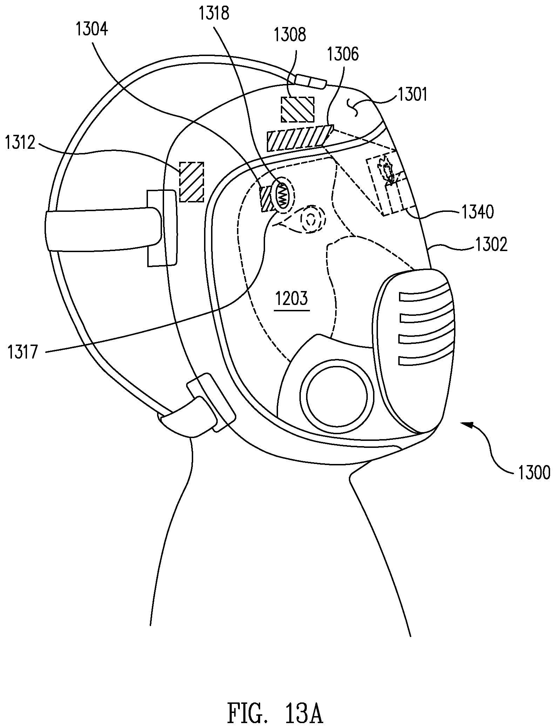

Figs. 13A-C illustrate various views of wearable apparatus 1200 implemented as a self-contained breathing apparatus (SCBA) 1300, in accordance with embodiments of the disclosure. In particular, Figs. 13A and 13B illustrate side and front views, respectively, of SCBA 1300 worn by user 1203 and having infrared imaging modules 1304 and a projector 1306 in accordance with embodiments of the disclosure. Fig. 13C illustrates a cross-sectional view of a window assembly 1318 of SCBA 1300, taken along line C-C' of Fig. 13B in accordance with an embodiment of the disclosure.

In one embodiment, SCBA 1300 may be implemented as a face mask for use by firefighters and/or other emergency personnel working in hazardous environments. In this regard, SCBA 1300 may be implemented to attach to a portable air supply (e.g., one or more high-pressure air tanks) and may further include an inhalation connection (e.g., a mouthpiece or orinasal cover and a hose connector) to the air supply while protecting a wearer's face from hazardous environments. In another embodiment, SCBA 1300 may be configured for underwater use as a self-contained underwater breathing apparatus (SCUBA).

SCBA 1300 may include a shield 1302, one or more infrared imaging modules 1304, projector 1306, a processor 1308, and a communication module 1312, all of which may be implemented in the same or similar manner as various corresponding components of wearable apparatus 1200 described above with regard to Fig. 12.

SCBA 1300 may further include a mask frame 1301 (e.g., corresponding to structural members 1201 of Fig. 12) onto which shield 1302 is sealingly fit. Mask frame 1301 may include an edge that compliantly engages the contours of the user's face, so that the user's face and SCBA 1300 form an interior space that is substantially sealed from an external environment. In some examples, the interior space of SCBA 1300 may maintain a positive pressure (i.e., higher pressure inside SCBA 1300 than outside) so as to prevent inward leaking.

Shield 1302 may be made of a clear polymer (e.g., polycarbonate) or other similar suitable materials that allow user 1203 to see through while providing a protective barrier against heat, flames, intense infrared and ultraviolet rays, debris, and/or other harmful elements from an external environment. Shield 1302 may comprise multiple layers of protective shields and/or surface coatings to enhance protection.

One or more infrared imaging modules 1304 may be positioned behind shield 1302 or mask frame 1301, and internal to SCBA 1300. Similarly, projector 1306, processor 1308, communication module 1312, and other components may be placed internal to SCBA 1300 (e.g., behind shield 1302 and behind or within mask frame 1301). It will be appreciated that these various components are thus advantageously protected by shield 1302 and mask frame 1301 of SCBA 1300 itself, without the need for separate protective external housings. It will also be appreciated that the small size and weight of infrared imaging modules 1304 advantageously permit these components to be positioned internal to SCBA 1300. In contrast, conventional infrared cameras are typically too bulky and heavy for such placement and thus must be implemented with separate protective housings that add even further weight and bulk, making them unsuitable for an apparatus worn on the head or face of a user. In another example, one or more infrared imaging modules 1304 may be positioned on or attached to SCBA 1300 externally.

Shield 1302 may include one or more apertures 1317 sealed by corresponding window assemblies 1318 capable of passing infrared radiation through to corresponding infrared imaging modules 1304 situated behind shield 1302, behind mask frame 1301, and/or within mask frame 1301.

In one embodiment, such a window assembly 1318 may include a window 1319 and a frame 1320. Window 1319 may be configured to pass infrared radiation. For example, window 1319 may include silicon and/or other materials where appropriate to pass infrared radiation. Frame 1320 may be configured to hold window 1319 and seal aperture 1317 with window 1319. In another example, window assembly 1318 may be implemented by window 1319 alone without frame 1320. In another example, infrared imaging module 1304 may be configured to seal aperture 1317 to prevent user 1203 from being exposed to the external environment if the seal provided by the window assembly 1318 fails (e.g., to prevent inward leaking of gas, liquid, radiation, and/or other elements into the interior of SCBA 1300).

As discussed above in connection with projector 1206 of Fig. 12, projector 1306 may allow user 1203 to selectively turn on/off and adjust the position of a projected user-viewable thermal image 1340, for example, for simultaneously viewing projected user-viewable thermal image 1340 and an external environment through shield 1302. Distortion correction may be performed by projector 1306 and/or processor 1308 for user-viewable thermal image 1340 to be projected on a curved surface of shield 1302 and/or projected at an oblique angle, as described above. In other examples, projector 1306 may project user-viewable thermal image 1340 on an outer surface of shield 1302 and/or a plated portion of a surface of shield 1302.

Thus, it will be appreciated that SCBA 1300 advantageously allows user 1203 (e.g., firefighters, emergency personnel, divers, or anyone wearing SCBA 1300 for protection from an external environment) to comfortably view a user-viewable thermal image 1340 that helps user 1203 to recognize much more about an external environment (e.g., see through smoke, water or in darkness, discern victims or other objects, detect the base of fire, detect the temperature of objects, detect invisible gas leaks, or other phenomena) than what can be seen through the naked eye or through CCD-based or CMOS-based sensors, while also protecting the user's face and various components (e.g., protecting infrared imaging modules 1304, projector 1306, processor 1308 without a need for bulky and heavy external protective housings).

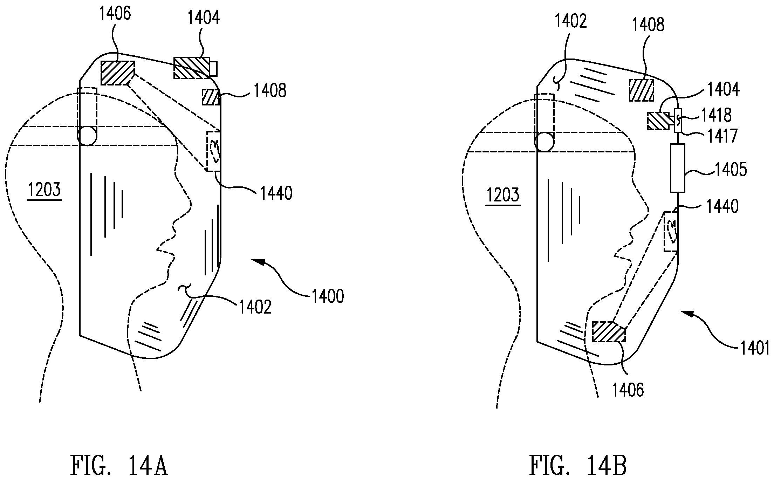

Figs. 14A-B illustrate side views of wearable apparatus 1200 implemented as welding masks 1400 and 1401 in accordance with various embodiments of the disclosure. Welding masks 1400/1401 may be worn by user 1203 and may include a shield 1402, one or more infrared imaging modules 1404, a projector 1406, and a processor 1408, all of which may be implemented in the same or similar manner as various corresponding components of wearable apparatus 1200 and SCBA 1300 described above. In welding mask 1400 of Fig. 14A, projector 1406 is top mounted and shield 1402 has no user viewable opening. In welding mask 1401 of Fig. 14B, projector 1406 is bottom mounted and shield 1402 has a user viewable opening 1405.

Shield 1402 may be made of one or more layers of durable material that is opaque or substantially nontransparent, so as to protect a user's face from intense light (e.g., including infrared and ultraviolet light) as well as from heat, sparks, and other debris that may be generated during welding. In the embodiment of Fig. 14B, viewing window 1405 may be tinted (e.g., using tinted sheets of glass, polarized lenses, automatic LCD shutter, or other appropriately tinted materials) to attenuate the intensity of light that may reach user's eyes while still allowing user 1203 to see through.

As described above in connection with Figs. 12 and 13A-C, various components may be protected by shield 1402 of welding masks 1400/1401. As shown in Fig. 14B, shield 1402 may include one or more apertures 1417 sealed with corresponding window assemblies 1418 implemented in the same or similar manner as window assemblies 1218/1318 described above, so as to pass infrared light through to corresponding infrared imaging modules 1404 that may be positioned internal to shield 1402. In one example, one or more infrared imaging modules 1404 may be mounted externally, for example on a top outside surface of shield 1402, as shown in Fig. 14A.

Projector 1406 may be configured to project a user-viewable thermal image 1440 of a welding environment on a portion of a surface of shield 1402 that is comfortable for a user to view, as shown in Figs. 14A-B. It is to be understood that a surface of shield 1402 may also include a surface of viewing window 1405, if desired.

User-viewable thermal image 1440 may help user 1203 better discern a welding scene, since user-viewable thermal images 1440 may be substantially clear of blindingly intense visible light radiation that are generated when welding. As described above with respect to Fig. 12, infrared imaging modules 1404 and/or processor 1408 may be configured to provide automatic exposure control, so as to generate user-viewable thermal images 1440 that are desirably adjusted to the infrared intensity and temperature level in the welding scene. Exposure-adjusted user-viewable thermal images 1440 may provide a clear view of the welding scene even when the infrared intensity and temperature level change (e.g., when user 1203 turns off a welding arc to adjust a welding tip of appropriate welding equipment), so that user 1203 can view the welding scene without having to lift, remove, and/or otherwise adjust welding mask 1400/1401 for a better view.

In addition, processor 1408 may be configured to overlay temperature readings and/or temperature scales onto a user-viewable thermal image 1440 to be projected onto a surface of shield 1402. Such temperature readings and/or temperature scales may help a user to determine whether the temperature of a welding arc and/or welding pool is proper for a welding task.

Thus, it will be appreciated that welding masks 1400/1401 advantageously protect a welder's face as well as various components of welding masks 1400/1401 from harsh elements of a welding environment, while also presenting to a welder a user-viewable thermal image 1440 that provides a clearer view of a welding environment along with useful information such as temperature readings of weld materials and a view of otherwise invisible objects (e.g., gas leaks).

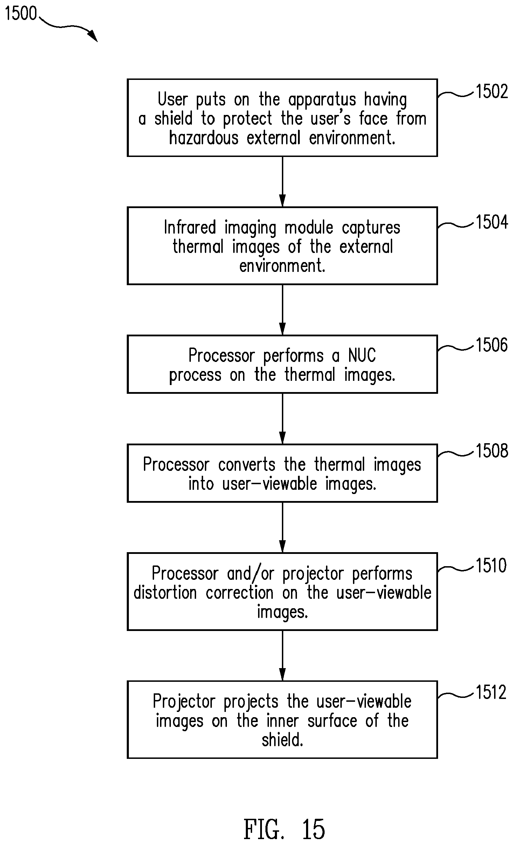

Fig. 15 illustrates a process to present a user-viewable thermal image on wearable apparatus 1200, in accordance with an embodiment of the disclosure. In this regard, the process of Fig. 15 may be applied generally to wearable apparatus 1200 and also to particular examples of wearable apparatus 1200 such as SCBA 1300, welding masks 1400/1401, and other applications where appropriate.

At block 1502, user 1203 may put on wearable apparatus 1200 having shield 1202/1302/1402 that is configured to protect at least a portion of the user's face from external environment 1230. For example, a firefighter may put on SCBA 1300 to protect the firefighter's face from a scene of fire, a diver may put on SCBA 1300 when diving underwater, or a welder may put on welding mask 1400/1401 to protect the welder's face from a hazardous welding environment.

At block 1504, one or more thermal images of external environment 1230 may be captured by one or more infrared imaging modules 1204/1304/1404. The one or more thermal images may be received, for example, at processor 1208/1308/1408 that is communicatively coupled via wired or wireless link to one or more infrared imaging modules 1204/1304/1404. At block 1506, a NUC process may be performed to remove noise from the thermal images, for example, by using various NUC techniques disclosed herein.

From the thermal images, user-viewable images (e.g., thermograms) may be generated by processor 1208/1308/1408 at block 1508. Also at block 1508, in various embodiments, additional information and/or alarms may be overlaid onto the user-viewable images by processor 1208/1308/1408. Also at block 1508, if processor 1208/1308/1408 is configured to receive one or more thermal images from two or more infrared imaging modules 1204/1304/1404, stereoscopic user-viewable images of external environment 1230 may be generated by processor 1208/1308/1408.

At block 1510, a distortion correction may be performed on the user-viewable images by projector 1206/1306/1406 and/or processor 1208/1308/1408. For example, projector 1206/1306/1406 may optically correct distortion of the user-viewable images to be projected onto a curved surface and/or projected at an oblique angle. In another example, projector 1206/1306/1406 and/or processor 1208/1308/1408 may correct such distortion computationally using appropriate holographic processes for projector 1206/1306/1406 based on interference/diffraction patterns.

At block 1512, the corrected user-viewable images may be projected onto a surface of shield 1202/1302/1402 by projector 1206/1306/1406 for viewing by a user while wearing wearable apparatus 1200/SCBA 1300/welding mask 1400. The user-viewable images may be projected onto an area of shield 1202/1302/1402 that is comfortable for viewing by a user, for example, for simultaneously viewing projected images and an external environment through shield 1202/1302/1402.

Where applicable, various embodiments provided by the present disclosure can be implemented using hardware, software, or combinations of hardware and software. Also where applicable, the various hardware components and/or software components set forth herein can be combined into composite components comprising software, hardware, and/or both without departing from the scope of the appended claims. Where applicable, the various hardware components and/or software components set forth herein can be separated into sub-components comprising software, hardware, or both without departing from the scope of the appended claims. In addition, where applicable, it is contemplated that software components can be implemented as hardware components, and vice-versa.

Software in accordance with the present disclosure, such as non-transitory instructions, program code, and/or data, can be stored on one or more non-transitory machine readable mediums. It is also contemplated that software identified herein can be implemented using one or more general purpose or specific purpose computers and/or computer systems, networked and/or otherwise. Where applicable, the ordering of various steps described herein can be changed, combined into composite steps, and/or separated into sub-steps to provide features described herein.

Embodiments described above illustrate but do not limit the invention. It should also be understood that numerous modifications and variations are possible in accordance with the scope of the invention. Accordingly, the scope of the invention is defined only by the following claims.

## Claims

1. A wearable apparatus (1200) comprising:
a shield (1202,1302,1402) configured to protect at least a portion of a user's face from an external environment;
an infrared imaging module (1204,1304,1404) comprising a focal plane array (FPA) configured to capture a thermal image of the external environment;
an aperture (1217,1317,1417) in the shield (1202,1302,1402);
a window assembly (1218,1318,1418) configured to seal the aperture (1217,1317,1417) and pass infrared radiation from the external environment to the infrared imaging module (1204,1304,1404);
a processor (1208,1308,1408) configured to convert the thermal image into a user-viewable image of the external environment; and
a projector (1206,1306,1406) configured to project the user-viewable image onto an inner surface of the shield (1202,1302,1402) for viewing by the user while wearing the apparatus (1200),
wherein the infrared imaging module (1204,1304,1404) and the projector (1206,1306,1406) are positioned interior to and behind said shield (1202,1302,1402) to protect the infrared imaging module (1204,1304,1404) and the projector (1206,1306,1406) from the external environment

2. The apparatus (1200) of claim 1, wherein the window assembly (1218,1318,1418) comprises:
a window (1319) comprising silicon and configured to pass the infrared radiation, and
a frame (1320) configured to hold the window (1319) and seal the aperture (1217,1317,1417) with the window; and
the infrared imaging module (1204,1304,1404) is configured to seal the aperture (1217,1317,1417) to prevent the user from being exposed to the external environment if the seal provided by the window assembly (1218,1318,1418) fails.

3. The apparatus (1200) of claim 1, wherein:
the apparatus (1200) is a self-contained breathing apparatus (SCBA) further comprising a mask frame (1301) sealingly coupled to the shield (1202,1302,1402) and configured to sealingly engage the user; and
the shield (1202,1302,1402) is configured to pass at least some visible light from the external environment to the user for viewing the external environment through the shield (1202,1302,1402).

4. The apparatus (1200) of claim 1, wherein:
the apparatus (1200) is a welding mask; and
at least a majority of the shield (1202,1302,1402) is substantially opaque and configured to substantially block visible light from the external environment.

5. The apparatus (1200) of claim 1, wherein:
the infrared imaging module (1204,1304,1404) is a first infrared imaging module;
the thermal image is a first thermal image;
the apparatus (1200) further comprises a second infrared imaging module configured to capture a second thermal image of the external environment; and
the user-viewable image is a stereoscopic image of the external environment based on the first and second thermal images.

6. The apparatus (1200) of claim 1, wherein the projector (1206,1306,1406) is a holographic projector.

7. A method (1500) of presenting a user-viewable image on a wearable apparatus (1200), the method comprising:
receiving infrared radiation from an external environment through an aperture in a shield of the wearable apparatus and through a window assembly configured to seal the aperture;
capturing (1504), at a focal plane array (FPA) of an infrared imaging module of the wearable apparatus, a thermal image of the external environment;
converting (1508) the thermal image into a user-viewable image of the external environment;
projecting (1512) the user-viewable image by a projector onto an inner surface of the shield for viewing by a user; and
protecting (1502) at least a portion of the user's face, the infrared imaging module, and the projector from the external environment by said shield, wherein the infrared imaging module and the projector are positioned interior to and behind the shield to be protected by the shield.

8. The method of claim 7, further comprising sealing the aperture by the infrared imaging module to prevent the user from being exposed to the external environment if the seal provided by the window assembly fails, wherein the window assembly comprises:
a window comprising silicon and configured to pass the infrared radiation; and a frame configured to hold the window and seal the aperture with the window.

9. The method of claim 7, wherein the apparatus is a self-contained breathing apparatus (SCBA), the method further comprising:
sealingly engaging a mask frame of the apparatus with the user, wherein the mask frame is sealingly coupled to the shield;
passing at least some visible light from the external environment through the shield to the user for viewing the external environment through the shield.

10. The method of claim 7, wherein the apparatus is a welding mask and at least a majority of the shield is substantially opaque, the method further comprising substantially blocking visible light from the external environment by the shield.

11. The method of claim 7, wherein:
the infrared imaging module is a first infrared imaging module;
the thermal image is a first thermal image;
the method further comprises capturing, at a second infrared imaging module of the wearable apparatus, a second thermal image of the external environment; and
the user-viewable image is a stereoscopic image of the external environment based on the first and second thermal images.

12. A method of constructing a wearable apparatus (1200), the method comprising:
providing a shield (1202, 1302, 1402) for protecting at least a portion of a user's face from an external environment;
providing an aperture (1217, 1317, 1417) in the shield;
positioning an infrared imaging module (1204, 1304, 1404) interior to and behind the shield to be protected from the external environment by the shield without a need for separate external protective housing, wherein the infrared imaging module comprises a focal plane array (FPA) configured to capture a thermal image of the external environment while the apparatus is worn by the user;
sealing the aperture with a window assembly (1218, 1318, 1418) that is adapted to pass infrared radiation from the external environment to the infrared imaging module;
positioning a projector (1206, 1306, 1406) interior to and behind said shield to be protected from the external environment by the shield wherein the projector is configured to project a user-viewable image of the external environment onto an inner surface of the shield for viewing by the user while wearing the apparatus; and
communicatively coupling a processor (1208, 1308, 1408) with the infrared imaging module and the projector.

## Patentansprüche

1. Tragbare Vorrichtung (1200), umfassend:
eine Abschirmung (1202, 1302, 1402), die konfiguriert ist, um mindestens einen Abschnitt des Gesichts eines Benutzers vor einer äußeren Umgebung zu schützen;
ein Infrarotbildgebungsmodul (1204, 1304, 1404), umfassend eine Brennebenenanordnung (FPA), die konfiguriert ist, um ein Wärmebild der äußeren Umgebung aufzunehmen;
eine Apertur (1217, 1317, 1417) in der Abschirmung (1202, 1302, 1402);
einen Scheibenaufbau (1218, 1318, 1418), der konfiguriert ist, um die Apertur (1217, 1317, 1417) abzudichten und Infrarotstrahlung von der äußeren Umgebung zu dem Infrarotbildgebungsmodul (1204, 1304, 1404) durchzulassen;
einen Prozessor (1208, 1308, 1408), der konfiguriert ist, um das Wärmebild in ein vom Benutzer sichtbares Bild der äußeren Umgebung umzuwandeln; und
einen Projektor (1206, 1306, 1406), der konfiguriert ist, um das vom Benutzer sichtbare Bild auf eine innere Oberfläche der Abschirmung (1202, 1302, 1402) zu projizieren, für das Betrachten durch den Benutzer, während er die Vorrichtung (1200) trägt,
wobei das Infrarotbildgebungsmodul (1204, 1304, 1404) und der Projektor (1206, 1306, 1406) innerhalb und hinter der Abschirmung (1202, 1302, 1402) positioniert ist, um das Infrarotbildgebungsmodul (1204, 1304, 1404) und den Projektor (1206, 1306, 1406) vor der äußeren Umgebung zu schützen.

2. Vorrichtung (1200) gemäß Anspruch 1, wobei der Scheibenaufbau (1218, 1318, 1418) umfasst:
eine Scheibe (1319), umfassend Silizium, und konfiguriert, die Infrarotstrahlung durchzulassen, und
ein Rahmen (1320), das konfiguriert ist, die Scheibe (1319) zu halten und die Apertur (1217, 1317, 1417) mit der Scheibe abzudichten; und
das Infrarotbildgebungsmodul (1204, 1304, 1404) ist konfiguriert, die Apertur (1217, 1317, 1417) abzudichten, um zu verhindern, dass der Benutzer der äußeren Umgebung ausgesetzt wird, wenn die durch den Scheibenaufbau (1218, 1318, 1418) bereitgestellte Abdichtung versagt.

3. Vorrichtung (1200) gemäß Anspruch 1, wobei:
die Vorrichtung (1200) eine unabhängige Atemvorrichtung (SCBA) ist, weiterhin umfassend einen Maskenrahmen (1301), der abdichtend mit der Abschirmung (1202, 1302, 1402) verbunden ist und konfiguriert ist, den Benutzer abdichtend zu erfassen; und
die Abschirmung (1202, 1302, 1402) konfiguriert ist, mindestens etwas sichtbares Licht von der äußeren Umgebung zum Benutzer durchzulassen, um die äußere Umgebung durch die Abschirmung (1202, 1302, 1402) zu betrachten.

4. Vorrichtung (1200) gemäß Anspruch 1, wobei:
die Vorrichtung (1200) eine Schweißmaske ist; und
mindestens ein Großteil der Abschirmung (1202, 1302, 1402) im Wesentlichen lichtundurchlässig ist und konfiguriert ist, sichtbares Licht von der äußeren Umgebung im Wesentlichen zu blockieren.

5. Vorrichtung (1200) gemäß Anspruch 1, wobei:
das Infrarotbildgebungsmodul (1204, 1304, 1404) ein erstes Infrarotbildgebungsmodul ist;
das Wärmebild ein erstes Wärmebild ist;
die Vorrichtung (1200) weiterhin ein zweites Infrarotbildgebungsmodul umfasst, das konfiguriert ist, ein zweites Wärmebild der äußeren Umgebung aufzunehmen; und
das vom Benutzer sichtbare Bild ist ein räumliches Bild der äußeren Umgebung auf der Grundlage des ersten und des zweiten Wärmebildes.

6. Vorrichtung (1200) gemäß Anspruch 1, wobei der Projektor (1206, 1306, 1406) ein holographischer Projektor ist.

7. Verfahren (1500) zum Darstellen eines vom Benutzer sichtbaren Bildes auf einer tragbaren Vorrichtung (1200), wobei das Verfahren umfasst:
Erhalten von Infrarotstrahlung von einer äußeren Umgebung durch eine Apertur in einer Abschirmung der tragbaren Vorrichtung und durch einen Scheibenaufbau, der konfiguriert wird, die Apertur abzudichten;
Erfassen (1504) eines Wärmebildes von der äußeren Umgebung in einer Brennebenenanordnung (FPA) eines Infrarotbildgebungsmoduls der tragbaren Vorrichtung;
Umwandeln (1508) des Wärmebildes in ein vom Benutzer sichtbares Bild der äußeren Umgebung;
Projizieren (1512) des vom Benutzer sichtbaren Bildes durch einen Projektor auf eine innere Oberfläche der Abschirmung zum Betrachten durch einen Benutzer; und
Schützen (1502) mindestens eines Abschnitts des Gesichts des Benutzers, des Infrarotbildgebungsmoduls und des Projektors vor der äußeren Umgebung durch die Abschirmung, wobei das Infrarotbildgebungsmodul und der Projektor innerhalb und hinter der Abschirmung positioniert werden, um durch die Abschirmung geschützt zu werden.

8. Verfahren gemäß Anspruch 7, weiterhin umfassend Abdichten der Apertur durch das Infrarotbildgebungsmodul, um zu verhindern, dass der Benutzer der äußeren Umgebung ausgesetzt wird, wenn die durch den Scheibenaufbau bereitgestellte Abdichtung versagt, wobei der Scheibenaufbau umfasst:
eine Scheibe, umfassend Silizium, und konfiguriert, die Infrarotstrahlung durchzulassen, und
ein Rahmen, der konfiguriert wird, die Scheibe zu halten und die Apertur mit der Scheibe abzudichten.

9. Verfahren gemäß Anspruch 7, wobei die Vorrichtung eine unabhängige Atemvorrichtung (SCBA) ist, wobei das Verfahren weiterhin umfasst:
abdichtendes Erfassen eines Maskenrahmens der Vorrichtung mit dem Benutzer, wobei der Maskenrahmen abdichtend mit der Abschirmung verbunden wird;
Durchlassen mindestens etwas sichtbares Licht von der äußeren Umgebung durch die Abschirmung zum Benutzer, um die äußere Umgebung durch die Abschirmung zu betrachten.

10. Verfahren gemäß Anspruch 7, wobei die Vorrichtung eine Schweißmaske ist und mindestens ein Großteil der Abschirmung ist im Wesentlichen lichtundurchlässig, wobei das Verfahren weiterhin im Wesentlichen Blockieren sichtbares Lichts von der äußeren Umgebung durch die Abschirmung umfasst.

11. Verfahren gemäß Anspruch 7, wobei:
das Infrarotbildgebungsmodul ein erstes Infrarotbildgebungsmodul ist;
das Wärmebild ein erstes Wärmebild ist;
das Verfahren weiterhin umfasst, Erfassen eines zweiten Wärmebildes von der äußeren Umgebung an einem zweiten Infrarotbildgebungsmodul der tragbaren Vorrichtung; und
das vom Benutzer sichtbare Bild ist ein räumliches Bild der äußeren Umgebung auf der Grundlage des ersten und des zweiten Wärmebildes.

12. Verfahren zum Herstellen einer tragbaren Vorrichtung (1200), wobei das Verfahren umfasst:
Bereitstellen einer Abschirmung (1202, 1302, 1402), um mindestens einen Abschnitt des Gesichts eines Benutzers vor einer äußeren Umgebung zu schützen;
Bereitstellen einer Apertur (1217, 1317, 1417) in der Abschirmung;
Positionieren eines Infrarotbildgebungsmoduls (1204, 1304, 1404) innerhalb und hinter der Abschirmung, um vor der äußeren Umgebung durch die Abschirmung geschützt zu werden ohne eine Notwendigkeit für separates äußeres Schutzgehäuse, wobei das Infrarotbildgebungsmodul eine Brennebenenanordnung (FPA) umfasst, die konfiguriert wird, ein Wärmebild der äußeren Umgebung aufzunehmen, während die Vorrichtung durch den Benutzer getragen wird;
Abdichten der Apertur mit einem Scheibenaufbau (1218, 1318, 1418), der angepasst wird, Infrarotstrahlung von der äußeren Umgebung zu dem Infrarotbildgebungsmodul durchzulassen;
Positionieren eines Projektors (1206, 1306, 1406) innerhalb und hinter der Abschirmung, um vor der äußeren Umgebung durch die Abschirmung geschützt zu werden, wobei der Projektor konfiguriert wird, ein vom Benutzer sichtbares Bild von der äußeren Umgebung auf eine innere Oberfläche der Abschirmung zu projizieren, um durch den Benutzer während Tragens der Vorrichtung betrachtet zu werden; und
kommunizierendes Verbinden eines Prozessors (1208, 1308, 1408) mit dem Infrarotbildgebungsmodul und dem Projektor.

## Revendications

1. Un appareil portable (1200) comprenant :
un écran (1202, 1302, 1402) configuré pour protéger au moins une partie du visage d'un utilisateur contre un environnement externe ;
un module d'imagerie infrarouge (1204, 1304, 1404) comprenant une matrice plan focal (FPA) configurée pour capturer une image thermique de l'environnement externe ;
une ouverture (1217, 1317, 1417) dans l'écran (1202, 1302, 1402) ;
un ensemble formant fenêtre (1218, 1318, 1418) configuré pour clore l'ouverture (1217, 1317, 1417) de façon étanche et laisser passer le rayonnement infrarouge provenant de l'environnement extérieur au module d'imagerie infrarouge (1204, 1304, 1404);
un processeur (1208, 1308, 1408) configuré pour convertir l'image thermique en une image de l'environnement externe visualisable par un utilisateur ; et
un projecteur (1206, 1306, 1406) configuré pour projeter l'image visualisable par un utilisateur sur une surface intérieure de l'écran (1202, 1302, 1402) pour que l'utilisateur puisse la visualiser tout en portant l'appareil (1200),
le module d'imagerie infrarouge (1204, 1304, 1404) et le projecteur (1206, 1306, 1406) étant disposés à l'intérieur et derrière ledit écran (1202, 1302, 1402), pour protéger le module d'imagerie infrarouge (1204, 1304, 1404) et le projecteur (1206, 1306, 1406) contre l'environnement externe.

2. L'appareil (1200) selon la revendication 1, dans lequel l'ensemble formant fenêtre (1218, 1318, 1418) comprend :
une fenêtre (1319) comprenant du silicium et configurée pour laisser passer le rayonnement infrarouge, et
un encadrement (1320) configuré pour maintenir la fenêtre (1319) et fermer l'ouverture (1217, 1317, 1417) de façon étanche avec la fenêtre ; et
le module d'imagerie infrarouge (1204, 1304, 1404) est configuré pour clore l'ouverture (1217, 1317, 1417) de façon étanche afin d'empêcher que l'utilisateur soit exposé à l'environnement externe si l'étanchéité fournie par l'ensemble formant de fenêtre (1218, 1318, 1418) devient défaillante.

3. L'appareil (1200) selon la revendication 1, dans lequel :
l'appareil (1200) est un appareil respiratoire autonome (SCBA) comprenant en outre un structure de masque (1301) relié de manière étanche à l'écran (1202, 1302, 1402) et configuré pour venir en engagement avec l'utilisateur de manière étanche ; et
l'écran (1202, 1302, 1402) est configuré pour laisser passer au moins une partie de la lumière visible provenant de l'environnement externe vers l'utilisateur pour visualiser l'environnement externe à travers l'écran (1202, 1302, 1402).

4. L'appareil (1200) selon la revendication 1, dans lequel :
l'appareil (1200) est un masque de soudage ; et
au moins une majorité de l'écran (1202, 1302, 1402) est sensiblement opaque et configuré pour bloquer substantiellement la lumière visible provenant de l'environnement externe.

5. L'appareil (1200) selon la revendication 1, dans lequel :
le module d'imagerie infrarouge (1204, 1304, 1404) est un premier module d'imagerie infrarouge ;
l'image thermique est une première image thermique ;
l'appareil (1200) comprend en outre un deuxième module d'imagerie infrarouge configuré pour capturer une deuxième image thermique de l'environnement externe ; et
l'image visualisable par un utilisateur est une image stéréoscopique de l'environnement externe basée sur les première et deuxième images thermiques.

6. L'appareil (1200) selon la revendication 1, dans lequel le projecteur (1206, 1306, 1406) est un projecteur holographique.

7. Un procédé (1500) de présentation d'une image visualisable par un utilisateur sur un appareil portable (1200), le procédé comprenant :
le fait de recevoir un rayonnement infrarouge provenant d'un environnement externe à travers une ouverture dans un écran de l'appareil portable et à travers un ensemble formant fenêtre configuré pour clore l'ouverture de façon étanche ;
le fait (1504) de capturer, sur une matrice plan focal (FPA) d'un module d'imagerie infrarouge de l'appareil portable, une image thermique de l'environnement externe ;
le fait (1508) de convertir l'image thermique en une image de l'environnement externe visualisable par un utilisateur ;
le fait (1512) de projeter l'image visualisable par un utilisateur au moyen d'un projecteur sur une surface intérieure de l'écran pour permettre à l'utilisateur de la voir ; et
le fait (1502) de protéger au moins une partie du visage de l'utilisateur, du module d'imagerie infrarouge et du projecteur de l'environnement externe par ledit écran, le module d'imagerie infrarouge et le projecteur étant placés à l'intérieur et derrière l'écran de façon à être protégés par l'écran.

8. Le procédé selon la revendication 7, comprenant en outre le fait de clore l'ouverture de façon étanche par le module d'imagerie infrarouge afin d'empêcher que l'utilisateur soit exposé à l'environnement externe si l'étanchéité fournie par l'ensemble formant fenêtre devient défaillant, l'ensemble formant fenêtre comprenant :
une fenêtre comprenant du silicium et configurée pour laisser passer le rayonnement infrarouge ; et un encadrement configuré pour maintenir la fenêtre et clore de façon étanche l'ouverture avec la fenêtre.

9. Le procédé selon la revendication 7, dans lequel l'appareil est un appareil respiratoire autonome (SCBA), le procédé comprenant en outre :
le fait de placer de manière étanche une structure de masque de l'appareil sur l'utilisateur, la structure de masque étant reliée de manière étanche à l'écran ;
le fait de laisser passer au moins une partie de la lumière visible provenant de l'environnement externe à travers l'écran vers l'utilisateur pour voir l'environnement externe à travers l'écran.

10. Le procédé selon la revendication 7, dans lequel l'appareil est un masque de soudage et au moins une majorité de l'écran est substantiellement opaque, le procédé comprenant en outre le fait de bloquer substantiellement, par l'écran, la lumière visible provenant de l'environnement externe.

11. Le procédé selon la revendication 7, dans lequel :
le module d'imagerie infrarouge est un premier module d'imagerie infrarouge ;
l'image thermique est une première image thermique ;
le procédé comprend en outre le fait de capturer, au niveau d'un deuxième module d'imagerie infrarouge de l'appareil portable, une deuxième image thermique de l'environnement externe ; et
l'image visualisable par un utilisateur est une image stéréoscopique de l'environnement externe basée sur les première et deuxième images thermiques.

12. Un procédé de construction d'un appareil portable (1200), le procédé comprenant :
le fait de fournir un écran (1202, 1302, 1402) pour protéger au moins une partie du visage d'un utilisateur contre un environnement externe ;
le fait de prévoir une ouverture (1217, 1317, 1417) dans l'écran ;
le fait de positionner un module d'imagerie infrarouge (1204, 1304, 1404) à l'intérieur et derrière l'écran de façon à être protégé de l'environnement externe par l'écran sans avoir besoin d'un boîtier de protection externe séparé, le module d'imagerie infrarouge comprenant un matrice plan focal (FPA) configuré pour saisir une image thermique de l'environnement externe lorsque l'appareil est porté par l'utilisateur ;
le fait de clore l'ouverture de façon étanche avec un ensemble formant fenêtre (1218, 1318, 1418) qui est adapté pour laisser passer le rayonnement infrarouge de l'environnement externe vers le module d'imagerie infrarouge ;
le fait de positionner un projecteur (1206, 1306, 1406) à l'intérieur et derrière ledit écran de façon à être protégé de l'environnement externe par l'écran, le projecteur étant configuré pour projeter une image visualisable par un utilisateur de l'environnement externe sur une surface intérieure de l'écran pour que l'utilisateur puisse la voir tout en portant l'appareil ; et
le fait de relier un processeur (1208, 1308, 1408) de façon communicante au module d'imagerie infrarouge et au projecteur.
